# FASCICULE DE BREVET EUROPEEN

(11) **EP 0 874 838 B1**
(45) Date de publication et mention de la délivrance du brevet: **16.08.2001**
(21) Numéro de dépôt: 96942432.4
(22) Date de dépôt: 19.12.1996
(51) Int. Cl.: C07D 305/14, A61K 31/335

(54) **NOUVEAUX TAXOIDES, LEUR PREPARATION ET LES COMPOSITIONS PHARMACEUTIQUES QUI LES CONTIENNENT**
NEUE TAXOIDE, HERSTELLUNG UND PHARMAZEUTISCHE ZUSAMMENSETZUNGEN DIESE ENTHALTEND
NOVEL TAXOIDS, PREPARATION THEREOF AND PHARMACEUTICAL COMPOSITIONS CONTAINING SAME

(30) Priorité: 22.12.1995 FR 9515380
(43) Date de publication de la demande: 04.11.1998
(73) Titulaire: Aventis Pharma S.A., 92160 Antony (FR)
(72) Inventeur: Bouchard, Hervé, F-94320 Thiais (FR); Commerçon, Alain, F-94400 Vitry-sur-Seine (FR)
(74) Mandataire: Le Pennec, Magali
(86) Numéro de dépôt international: FR9602030
(87) Numéro de publication internationale: WO9723472

(56) Documents cités:
- EP-A- 0 600 517
- FR-A- 2 698 871

## Description

La présente invention concerne de nouveaux taxoïdes de formule générale : 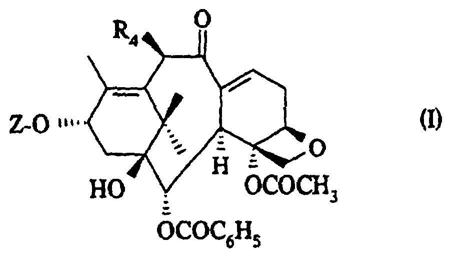 dans laquelle

Z représente un atome d'hydrogène ou un radical de formule générale : 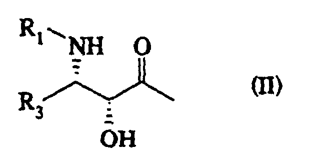 dans laquelle :
R₁ représente un radical benzoyle éventuellement substitué par un ou plusieurs atomes ou radicaux, identiques ou différents, choisis parmi les atomes d'halogène et les radicaux alcoyles contenant 1 à 4 atomes de carbone, alcoxy contenant 1 à 4 atomes de carbone ou trifluorométhyle, thénoyle ou furoyle ou un radical R₂-O-CO- dans lequel R₂ représente :
   - un radical alcoyle contenant 1 à 8 atomes de carbone, alcényle contenant 2 à 8 atomes de carbone, alcynyle contenant 3 à 8 atomes de carbone, cycloalcoyle contenant 3 à 6 atomes de carbone, cycloalcényle contenant 4 à 6 atomes de carbone, bicycloalcoyle contenant 7 à 10 atomes de carbone, ces radicaux étant éventuellement substitués par un ou plusieurs substituants choisis parmi les atomes d'halogène et les radicaux hydroxy, alcoxy contenant 1 à 4 atomes de carbone, dialcoylamino dont chaque partie alcoyle contient 1 à 4 atomes de carbone, pipéridino, morpholino, pipérazinyl-1 (éventuellement substitué en -4 par un radical alcoyle contenant 1 à 4 atomes de carbone ou par un radical phénylalcoyle dont la partie alcoyle contient 1 à 4 atomes de carbone), cycloalcoyle contenant 3 à 6 atomes de carbone, cycloalcényle contenant 4 à 6 atomes de carbone, phényle (éventuellement substitué par un ou plusieurs atomes ou radicaux choisis parmi les atomes d'halogène et les radicaux alcoyles contenant 1 à 4 atomes de carbone ou alcoxy contenant 1 à 4 atomes de carbone), cyano, carboxy ou alcoxycarbonyle dont la partie alcoyle contient 1 à 4 atomes de carbone,
   - un radical phényle ou α- ou β-naphtyle éventuellement substitué par un ou plusieurs atomes ou radicaux choisis parmi les atomes d'halogène et les radicaux alcoyles contenant 1 à 4 atomes de carbone ou alcoxy contenant 1 à 4 atomes de carbone ou un radical hétérocyclique aromatique à 5 chaînons choisi de préférence parmi les radicaux furyle et thiényle,
   - ou un radical hétérocyclyle saturé contenant 4 à 6 atomes de carbone éventuellement substitué par un ou plusieurs radicaux alcoyles contenant 1 à 4 atomes de carbone,
R₃ représente un radical alcoyle droit ou ramifié contenant 1 à 8 atomes de carbone, alcényle droit ou ramifié contenant 2 à 8 atomes de carbone, alcynyle droit ou ramifié contenant 2 à 8 atomes de carbone, cycloalcoyle contenant 3 à 6 atomes de carbone, phényle ou α- ou β-naphtyle éventuellement substitué par un ou plusieurs atomes ou radicaux choisis parmi les atomes d'halogène et les radicaux alcoyles, alcényles, alcynyles, aryles, aralcoyles, alcoxy, alcoylthio, aryloxy, arylthio, hydroxy, hydroxyalcoyle, mercapto, formyle, acyle, acylamino, aroylamino, alcoxycarbonylamino, amino, alcoylamino, dialcoylamino, carboxy, alcoxycarbonyle, carbamoyle, alcoylcarbamoyle, dialcoylcarbamoyle, cyano, nitro et trifluorométhyle, ou un hétérocycle aromatique ayant 5 chaînons et contenant un ou plusieurs hétéroatomes, identiques ou différents, choisis parmi les atomes d'azote, d'oxygène ou de soufre et éventuellement substitué par un ou plusieurs substituants, identiques ou différents, choisis parmi les atomes d'halogène et les radicaux alcoyles, aryles, amino, alcoylamino, dialcoylamino, alcoxycarbonylamino, acyle, arylcarbonyle, cyano, carboxy, carbamoyle, alcoylcarbamoyle, dialcoylcarbamoyle ou alcoxycarbonyle, étant entendu que, dans les substituants des radicaux phényle, α- ou β-naphtyle et hétérocyclyles aromatiques, les radicaux alcoyles et les portions alcoyles des autres radicaux contiennent 1 à 4 atomes de carbone et que les radicaux alcényles et alcynyles contiennent 2 à 8 atomes de carbone et que les radicaux aryles sont des radicaux phényles ou α- ou β-naphtyles,
R₄ représente un atome d'hydrogène ou un radical hydroxy ou un radical alcoxy contenant 1 à 6 atomes de carbone en chaîne droite ou ramifiée, alcényloxy contenant 3 à 6 atomes de carbone en chaîne droite ou ramifiée, alcynyloxy contenant 3 à 6 atomes de carbone en chaîne droite ou ramifiée, cycloalcoyloxy contenant 3 à 6 atomes de carbone, cycloalcényloxy contenant 3 à 6 atomes de carbone, alcanoyloxy dont la partie alcanoyle contient 1 à 6 atomes de carbone en chaîne droite ou ramifiée, alcénoyloxy dont la partie alcénoyle contient 3 à 6 atomes de carbone en chaîne droite ou ramifiée, alcynoyloxy dont la partie alcynoyle contient 3 à 6 atomes de carbone en chaîne droite ou ramifiée, cycloalcanoyloxy contenant 1 à 6 atomes de carbone, alcoxyacétyle dont la partie alcoyle contient 1 à 6 atomes de carbone en chaîne droite ou ramifiée, alcoylthioacétyle dont la partie alcoyle contient 1 à 6 atomes de carbone en chaîne droite ou ramifiée, alcoyloxycarbonyloxy dont la partie alcoyle contient 1 à 6 atomes de carbone en chaîne droite ou ramifiée, ces radicaux étant éventuellement substitués par un ou plusieurs atomes d'halogène ou par un radical alcoxy contenant 1 à 4 atomes de carbone, alcoylthio contenant 1 à 4 atomes de carbone, ou un radical carboxy, alcoyloxycarbonyle dont la partie alcoyle contient 1 à 4 atomes de carbone, cyano, carbamoyle, N-alcoylcarbamoyle ou N,N-dialcoylcarbamoyle dont chaque partie alcoyle contient 1 à 4 atomes de carbone ou forme avec l'atome d'azote auquel elle est liée un radical hétérocyclique saturé contenant 5 ou 6 chaînons et éventuellement un second hétéroatome choisi parmi les atomes d'oxygène, de soufre ou d'azote éventuellement substitué par un radical alcoyle contenant 1 à 4 atomes de carbone ou un radical phényle ou un radical phénylalcoyle dont la partie alcoyle contient 1 à 4 atomes de carbone, ou bien R₄ représente un radical carbamoyloxy. alcoylcarbamoyloxy dont la partie alcoyle contient 1 à 4 atomes de carbone, dialcoylcarbamoyloxy dont chaque partie alcoyle contient 1 à 4 atomes de carbone ou un radical benzoyloxy ou hétérocyclylcarbonyloxy dans lequel la partie hétérocyclique représente un hétérocycle aromatique 5 ou 6 chaînons contenant un ou plusieurs hétéroatomes choisis parmi les atomes d'oxygène, de soufre ou d'azote.

La demande de brevet EP 600517 décrit des dérivés du paclitaxel modifié en position 6 et 7 par la présence d'une double liaison ou de deux groupes hydroxyle.

La demande de brevet FR 2 698 871 décrit des taxoïdes modifiés en position 7-8 par la présence d'un motif cyclopropyle.

De préférence les radicaux aryles pouvant être représentés par R₃ sont des radicaux phényles ou α- ou β-naphtyles éventuellement substitués par un ou plusieurs atomes ou radicaux choisis parmi les atomes d'halogène (fluor, chlore, brome, iode) et les radicaux alcoyles, alcényles, alcynyles, aryles, arylalcoyles, alcoxy, alcoylthio, aryloxy, arylthio, hydroxy, hydroxyalcoyle, mercapto, formyle, acyle, acylamino, aroylamino, alcoxycarbonylamino, amino, alcoylamino, dialcoylamino, carboxy, alcoxycarbonyle, carbamoyle, dialcoylcarbamoyle, cyano, nitro et trifluoro-méthyle, étant entendu que les radicaux alcoyles et les portions alcoyles des autres radicaux contiennent 1 à 4 atomes de carbone, que les radicaux alcényles et alcynyles contiennent 2 à 8 atomes de carbone et que les radicaux aryles sont des radicaux phényles ou α- ou β-naphtyles.

De préférence les radicaux hétérocycliques pouvant être représentés par R₃ sont des radicaux hétérocycliques aromatiques ayant 5 chaînons et contenant un ou plusieurs atomes, identiques ou différents, choisis parmi les atomes d'azote, d'oxygène ou de soufre, éventuellement substitués par un ou plusieurs substituants, identiques ou différents, choisis parmi les atomes d'halogène (fluor, chlore, brome, iode) et les radicaux alcoyles contenant 1 à 4 atomes de carbone, aryles contenant 6 à 10 atomes de carbone, alcoxy contenant 1 à 4 atomes de carbone, aryloxy contenant 6 à 10 atomes de carbone, amino, alcoylamino contenant 1 à 4 atomes de carbone, dialcoylamino dont chaque partie alcoyle contient 1 à 4 atomes de carbone, acylamino dont la partie acyle contient 1 à 4 atomes de carbone, alcoxycarbonylamino contenant 1 à 4 atomes de carbone, acyle contenant 1 à 4 atomes de carbone, arylcarbonyle dont la partie aryle contient 6 à 10 atomes de carbone, cyano, carboxy, carbamoyle, alcoylcarbamoyle dont la partie alcoyle contient 1 à 4 atomes de carbone, dialcoylcarbamoyle dont chaque partie alcoyle contient 1 à 4 atomes de carbone ou alcoxycarbonyle dont la partie alcoxy contient 1 à 4 atomes de carbone.

De préférence le radical R₄ représente un radical hydroxy ou un radical alcoxy droit ou ramifié contenant 1 à 6 atomes de carbone ou un radical alcanoyloxy contenant 1 à 6 atomes de carbone éventuellement substitué par un radical méthoxy, éthoxy, méthyltio, éthylthio, un radicl carboxy, méthoxycarbonyle, éthoxycarbonyle, cyano, carbamoyle, N-méthyl-carbamoyle, N-éthylcarbamoyle, N,N-diméthylcarbamoyle, N,N-diéthylcarbamoyle, N-pyrrolidinocarbonyle ou N-pipéridinocarbonyle.

Plus particulièrement, la présente invention concerne les produits de formule générale (I) dans laquelle Z représente un atome d'hydrogène ou un radical de formule générale (II) dans laquelle R₁ représente un radical benzoyle ou un radical R₂-O-CO-dans lequel R₂ représente un radical tert-butyle et R₃ représente un radical alcoyle contenant 1 à 6 atomes de carbone, alcényle contenant 2 à 6 atomes de carbone, cycloalcoyle contenant 3 à 6 atomes de carbone, phényle éventuellement substitué par un ou plusieurs atomes ou radicaux, identiques ou différents choisis parmi les atomes d'halogène (fluor, chlore) et les radicaux alcoyles (méthyle), alcoxy (méthoxy), dialcoylamino (diméthylamino), acylamino (acétylamino), alcoxycarbonylamino (tert-butoxycarbonylamino) ou trifluorométhyle ou un radical furyle-2 ou -3, thiényle-2 ou -3 ou thiazolyle-2, -4 ou -5 et R₄ représente un radical hydroxy ou un radical alcoyloxy droit ou ramifié contenant 1 à 6 atomes de carbone ou un radical alcanoyloxy contenant 1 à 6 atomes de carbone.

Plus particulièrement encore, la présente invention concerne les produits de formule générale (I) dans laquelle Z représente un atome d'hydrogène ou un radical de formule générale (II) dans laquelle R₁ représente un radical benzoyle ou un radical R₂-O-CO- dans lequel R₂ représente un radical tert-butyle et R₃ représente un radical isobutyle, isobutényle, butényle, cyclohexyle, phényle, furyle-2, furyle-3, thiényle-2, thiényle-3, thiazolyle-2, thiazolyle-4 ou thiazolyle-5, R₄ représente un radical hydroxy, méthoxy ou acétoxy.

Les produits de formule générale (I) dans laquelle Z représente un radical de formule générale (II) présentent des propriétés antitumorales et antileucémiques remarquables.

Selon l'invention, les produit de formule générale (I) peuvent être obtenus par chauffage en présence d'un agent d'activation tel qu'un halogénure de métal alcalin (chlorure de sodium), ou un azoture de métal alcalin (azoture de sodium) ou un sel d'ammonium ou de silice d'un produit de formule générale: 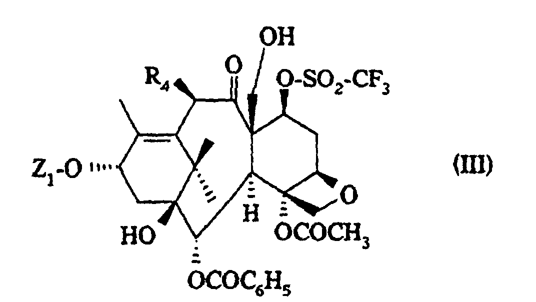 dans laquelle R₄ est défini comme précédemment et Z₁ représente un atome d'hydrogène ou un radical de formule générale: 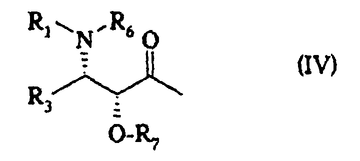 dans laquelle, ou bien, R₆ représente un atome d'hydrogène et R₇ représente un atome d'hydrogène ou un groupement protecteur de la fonction hydroxy, ou bien, R₆ et R₇ forment ensemble un hétérocycle saturé à 5 ou 6 chaînons, pour obtenir un produit de formule générale : 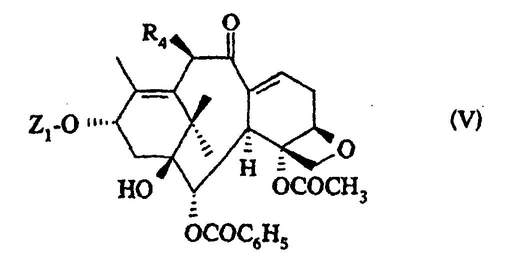 dans laquelle Z₁ et R₄ sont définis comme précédemment, suivi éventuellement du remplacement des groupements protecteurs par des atomes d'hydrogène.

Généralement, la réaction d'élimination est effectuée en opérant dans un solvant organique choisi parmi les éthers (tétrahydrofurane, diisopropyléther, méthyl t.butyléther), les nitriles (acétonitrile), les hydrocarbures aliphatiques halogénés (dichloroéthane) ou les esters aliphatiques (acétate d'éthyle) pris seuls ou en mélange à une température comprise entre 20°C et la température de reflux du mélange réactionnel.

De préférence, R₆ représente un atome d'hydrogène et R₇ représente un groupement protecteur de la fonction hydroxy ou bien R₆ et R₇ forment ensemble un hétérocycle saturé à 5 ou 6 chaînons.

Lorsque R₆ représente un atome d'hydrogène, R₇ représente de préférence un radical méthoxyméthyle, éthoxy-1 éthyle, benzyloxyméthyle, triméthylsilyle, triéthylsilyle, β-triméthylsilyléthoxyméthyle, benzyloxycarbonyle ou tétrahydropyrannyle.

Lorsque R₆ et R₇ forment ensemble un hétérocycle, celui-ci est de préférence un cycle oxazolidine éventuellement mono-substitué ou gem-disubstitué en position -2.

Le remplacement des groupements protecteurs R₇ et/ou R₆ et R₇ par des atomes d'hydrogène peut être effectué, selon leur nature de la manière suivante :
1) lorsque R₆ représente un atome d'hydrogène et R₇ représente un groupement protecteur de la fonction hydroxy, le remplacement des groupements protecteurs par des atomes d'hydrogène s'effectue au moyen d'un acide minéral (acide chlorhydrique, acide sulfurique, acide fluorhydrique) ou organique (acide acétique, acide méthanesulfonique, acide trifluorométhanesulfonique, acide p.toluènesulfonique) utilisé seul ou en mélange en opérant dans un solvant organique choisi parmi les alcools, les éthers, les esters, les hydrocarbures aliphatiques, les hydrocarbures aliphatiques halogénés, les hydrocarbures aromatiques ou les nitriles à une température comprise entre -10 et 60°C, ou au moyen d'une source d'ions fluorures tel qu'un complexe acide fluorhydrique-triéthylamine ou par hydrogénation catalytique,
2) lorsque R₆ et R₇ forment ensemble un hétérocycle saturé à 5 ou 6 chaînons et plus particulièrement un cycle oxazolidine de formule générale : 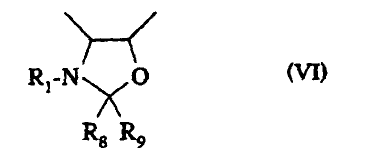 dans laquelle R₁ est défini comme précédemment, R₈ et R₉, identiques ou différents, représentent un atome d'hydrogène ou un radical alcoyle contenant 1 à 4 atomes de carbone, ou un radical aralcoyle dont la partie alcoyle contient 1 à 4 atomes de carbone et la partie aryle représente, de préférence, un radical phényle éventuellement substitué par un ou plusieurs radicaux alcoxy contenant 1 à 4 atomes de carbone, ou un radical aryle représentant, de préférence un radical phényle éventuellement substitué par un ou plusieurs radicaux alcoxy contenant 1 à 4 atomes de carbone, ou bien R₈ représente un radical alcoxy contenant 1 à 4 atomes de carbone ou un radical trihalométhyle tel que trichlorométhyle ou un radical phényle substitué par un radical trihalométhyle tel que trichlorométhyle et R₉ représente un atome d'hydrogène, ou bien R₈ et R₉ forment ensemble avec l'atome de carbone auquel ils sont liés un cycle ayant 4 à 7 chaînons, le remplacement du groupement protecteur formé par R₆ et R₇ par des atomes d'hydrogène peut être effectué, selon les significations de R₁, R₈ et R₉, de la manière suivante :
   a) lorsque R₁ représente un radical tert-butoxycarbonyle, R₈ et R₉, identiques ou différents, représentent un radical alcoyle ou un radical aralcoyle (benzyle) ou aryle (phényle), ou bien R₈ représente un radical trihalométhyle ou un radical phényle substitué par un radical trihalométhyle, et R₉ représente un atome d'hydrogène, ou bien R₈ et R₉ forment ensemble un cycle ayant de 4 à 7 chaînons, le traitement de l'ester de formule générale (V) par un acide minéral ou organique éventuellement dans un solvant organique tel qu'un alcool conduit au produit de formule générale: 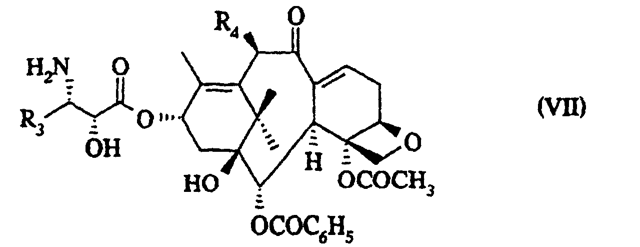 dans laquelle R₃ et R₄ sont définis comme précédemment, qui est acylé au moyen de chlorure de benzoyle dans lequel le noyau phényle est éventuellement substitué, de chlorure de thénoyle, de chlorure de furoyle ou d'un produit de formule générale:

      R₂-O-CO-X (VIII)

      dans laquelle R₂ est défini comme précédemment et X représente un atome d'halogène (fluor, chlore) ou un reste -O-R₂ ou -O-CO-O-R₂, pour obtenir un produit de formule générale (I) dans laquelle Z représente un radical de formule générale (II).
      De préférence, le produit de formule générale (V) est traité par l'acide formique à une température voisine de 20°C pour fournir le produit de formule générale (VII).
      De préférence, l'acylation du produit de formule générale (VII) au moyen d'un chlorure de benzoyle dans lequel le radical phényle est éventuellement substitué, de chlorure de thénoyle ou de chlorure de furoyle ou d'un produit de formule générale (VIII) est effectuée dans un solvant organique inerte choisi parmi les esters tels que l'acétate d'éthyle, l'acétate d'isopropyle ou l'acétate de n.butyle et les hydrocarbures aliphatiques halogénés tels que le dichlorométhane ou le dichloro-1,2 éthane en présence d'une base minérale telle que le bicarbonate de sodium ou organique telle que la triéthylamine. La réaction est effectuée à une température comprise entre 0 et 50°C, de préférence voisine de 20°C.
   b) lorsque R₁ représente un radical benzoyle éventuellement substitué, thénoyle ou furoyle ou un radical R₂O-CO- dans lequel R₂ est défini comme précédemment, R₈ représente un atome d'hydrogène ou un radical alcoxy contenant 1 à 4 atomes de carbone ou un radical phényle substitué par un ou plusieurs radicaux alcoxy contenant 1 à 4 atomes de carbone et R₉ représente un atome d'hydrogène, le remplacement du groupement protecteur formé par R₆ et R₇ par des atomes d'hydrogène s'effectue en présence d'un acide minéral (acide chlorhydrique, acide sulfurique) ou organique (acide acétique, acide méthanesulfonique, acide trifluorométhanesulfonique, acide p.toluènesulfonique) utilisé seul ou en mélange en quantité stoechiométrique ou catalytique, en operant dans un solvant organique choisi parmi les alcools, les éthers, les esters, les hydrocarbures aliphatiques, les hydrocarbures aliphatiques halogénés et les hydrocarbures aromatiques à une température comprise entre -10 et 60°C, de préférence entre 15 et 30°C.

Le produit de formule générale (III) dans laquelle R₄ représente un radical hydroxy peut être obtenu par action d'un d'un dérivé de l'acide trifluorométhanesulfonique tel que l'anhydride ou le N-phényl trifluorométhane-sulfonimide dans un solvant organique inerte tel qu'un hydrocarbure aliphatique éventuellement halogéné comme le dichlorométhane en opérant en présence d'une base organique telle que la pyridine ou une amine aliphatique tertiaire comme la triéthylamine à une température comprise entre -50 et 20°C sur un produit de formule générale : 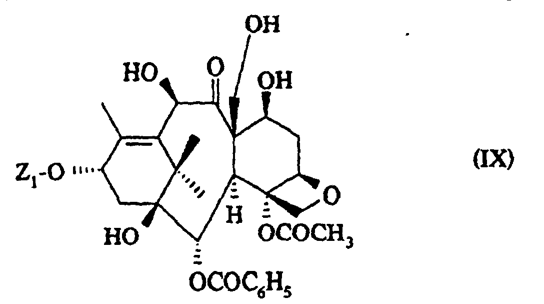 dans laquelle Z₁ est défini comme précédemment.

Le produit de formule générale (IX) peut être obtenu à partir d'un produit de formule générale : 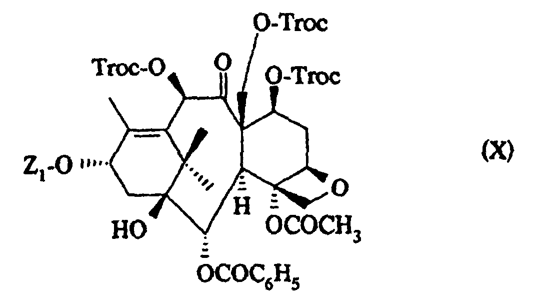 dans laquelle Z₁ est défini comme précédemment et Troc représente le radical trichlor-2,2,2-éthoxycarbonyle par action du zinc éventuellement associé à du cuivre en présence d'acide acétique à une température comprise entre 20 et 60°C ou au moyen d'un acide minéral ou organique tel que l'acide chlorhydrique ou l'acide acétique en solution dans un alcool aliphatique contenant 1 à 3 atomes de carbone ou dans un ester aliphatique tel que l'acétate d'éthyle, l'acétate d'isopropyle ou l'acétate de n.butyle en présence de zinc éventuellement associé à du cuivre.

Le produit de formule générale (X) dans laquelle Z₁ représente un radical de formule générale (IV) dans laquelle R₁ et R₃ sont définis comme précédemment, ou bien, R₆ représente un atome d'hydrogène et R₇ représente un groupement protecteur de la fonction hydroxy, ou bien, R₆ et R₇ forment ensemble un hétérocycle saturé à 5 ou 6 chainons, peut être obtenu par estérification du produit de formule : 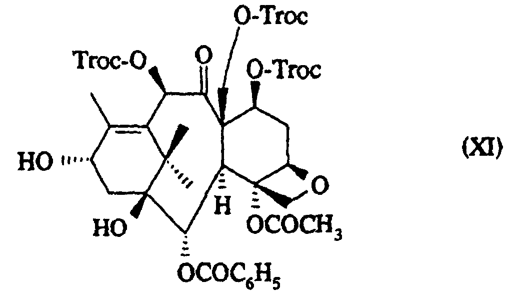 dans laquelle Troc est défini comme précédemment, au moyen d'un acide ou d'un dérivé de cet acide de formule générale: 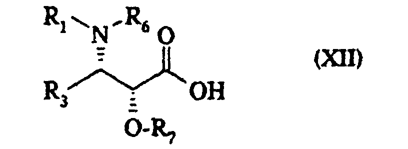 dans laquelle R₁ et R₃ sont définis comme précédemment et R₆ et R₇ sont définis comme ci-dessus.

L'estérification au moyen d'un acide de formule générale (XII) peut être effectuée en présence d'un agent de condensation (carbodiimide, carbonate réactif) et d'un agent d'activation (aminopyridines) dans un solvant organique (éther, ester, cétones, nitriles, hydrocarbures aliphatiques, hydrocarbures aliphatiques halogénés, hydrocarbures aromatiques) à une température comprise entre -10 et 90°C.

L'estérification peut aussi être réalisée en utilisant l'acide de formule générale (XII) sous forme d'anhydride symétrique en opérant en présence d'un agent d'activation (aminopyridines) dans un solvant organique (éthers, esters, cétones, nitriles, hydrocarbures aliphatiques, hydrocarbures aliphatiques halogénés, hydrocarbures aromatiques) à une température comprise entre 0 et 90°C.

L'estérification peut aussi être réalisée en utilisant l'acide de formule générale (XII) sous forme d'halogénure ou sous forme d'anhydride mixte avec un acide aliphatique ou aromatique, éventuellement préparé in situ, en présence d'une base (amine aliphatique tertiaire) en opérant dans un solvant organique (éthers, esters, cétones, nitriles, hydrocarbures aliphatiques, hydrocarbures aliphatiques halogénés, hydrocarbures aromatiques) à une température comprise entre 0 et 80°C.

Le produit de formule (XI) peut être obtenu dans les conditions décrites dans la demande internationale PCT WO 94/01425.

Le produit de formule générale (III) dans laquelle R₄ représente un radical alcoxy contenant 1 à 6 atomes de carbone en chaîne droite ou ramifiée, alcényloxy contenant 3 à 6 atomes de carbone en chaîne droite ou ramifiée, alcynyloxy contenant 3 à 6 atomes de carbone en chaîne droite ou ramifiée, cycloalcoyloxy contenant 3 à 6 atomes de carbone, cycloalcényloxy contenant 3 à 6 atomes de carbone, alcanoyloxy dont la partie alcanoyle contient 1 à 6 atomes de carbone en chaîne droite ou ramifiée, alcénoyloxy dont la partie alcénoyle contient 3 à 6 atomes de carbone en chaîne droite ou ramifiée, alcynoyloxy dont la partie alcynoyle contient 3 à 6 atomes de carbone en chaîne droite ou ramifiée, cycloalcanoyloxy contenant 1 à 6 atomes de carbone, alcoxyacétyle dont la partie alcoyle contient 1 à 6 atomes de carbone en chaîne droite ou ramifiée, alcoylthioacétyle dont la partie alcoyle contient 1 à 6 atomes de carbone en chaîne droite ou ramifiée, alcoyloxycarbonyloxy dont la partie alcoyle contient 1 à 6 atomes de carbone en chaîne droite ou ramifiée, ces radicaux étant éventuellement substitués par un ou plusieurs atomes d'halogène ou par un radical alcoxy contenant 1 à 4 atomes de carbone, alcoylthio contenant 1 à 4 atomes de carbone, ou un radical carboxy, alcoyloxycarbonyle dont la partie alcoyle contient 1 à 4 atomes de carbone, cyano, carbamoyle, N-alcoylcarbamoyle ou N,N-dialcoylcarbamoyle dont chaque partie alcoyle contient 1 à 4 atomes de carbone ou forme avec l'atome d'azote auquel elle est liée un radical hétérocyclique saturé contenant 5 ou 6 chaînons et éventuellement un second hétéroatome choisi parmi les atomes d'oxygène, de soufre ou d'azote éventuellement substitué par un radical alcoyle contenant 1 à 4 atomes de carbone ou un radical phényle ou un radical phénylalcoyle dont la partie alcoyle contient 1 à 4 atomes de carbone, ou bien R₄ représente un radical cabamoyloxy, alcoylcarbamoyloxy dont la partie alcoyle contient 1 à 4 atomes de carbone ou dialcoylcarbamoyloxy dont chaque partie alcoyle contient 1 à 4 atomes de carbone ou un radical benzoyloxy ou hétérocyclylcarbonyloxy dans lequel la partie hétérocyclique représente un hétérocycle aromatique 5 ou 6 chaînons contenant un ou plusieurs hétéroatomes choisis parmi les atomes d'oxygène, de soufre ou d'azote, et Z₁ est défini comme précédemment, peut être obtenu à partir d'un produit de formule générale (III) dans laquelle Z₁ est défini comme précédemment et R₄ représente un radical hydroxy, par action d'unproduit de formule générale :

R'₄-Y (XIII)

dans laquelle R'₄ est tel que R'₄-O- est identique à R₄ défini comme précédemment et Y repésente un groupe partant tel qu'un atome d'halogène ou un radical alcoylsulfonyle ou arylsulfonyle ou, lorsque R'₄ représente le reste d'un acide carboxylique, tel qu'un radical alcanoyloxy, un radical -O-R'₄.

Généralement, l'action d'un produit de formule générale (XIII) sur le produit de formule générale (III) défini ci-dessus est effectuée, après métallation éventuelle de la fonction hydroxy en 10 au moyen d'un hydrure de métal alcalin tel que l'hydrure de sodium, d'un amidure de métal alcalin tel que le diisopropylamidure de lithium ou d'un alcoylure de métal alcalin tel que le butyllithium, en opérant dans un solvant organique tel que le diméthylformamide ou le tetrahydrofurane à une température comprise entre -30 et 50°C, suivi éventuellement du remplacement du groupement protecteur de la fonction hydroxy dans les conditions décrites précédemment.

Le produit de formule générale (III) dans laquelle R₄ représente un atome d'hydrogène peut être obtenu à partir d'un produit de formule générale (III) dans laquelle R₄ représente un radical hydroxy dans les conditions décrites par exemple dans les demandes internationales PCT WO 93/06093 ou WO 94/11547 ou par tansformation du radical hydroxy représenté par R₄ en dithiocarbonate suivie de la réduction du produit obtenu au moyen d'un hydrure de trialcoylétain, ou encore par réduction en présence d'iodure de sammarium.

Les nouveaux produits de formule générale (I) obtenus par la mise en oeuvre des procédés selon l'invention peuvent être purifiés selon les méthodes connues telles que la cristallisation ou la chromatographie.

Les produits de formule générale (I) dans laquelle Z représente un radical de formule générale (II) présentent des propriétés biologiques remarquables.

In vitro, la mesure de l'activité biologique est effectuée sur la tubuline extraite de cerveau de porc par la méthode de M.L. Shelanski et coll., Proc. Natl. Acad. Sci. USA, 70, 765-768 (1973). L'étude de la dépolymérisation des microtubules en tubuline est effectuée selon la méthode de G. Chauvière et coll., C.R. Acad. Sci., 293, série II, 501-503 (1981). Dans cette étude les produits de formule générale (I) dans laquelle Z représente un radical de formule générale (II) se sont montrés au moins aussi actifs que le taxol et le Taxotère.

In vivo, les produits de formule générale (I) dans laquelle Z représente un radical de formule générale (II) se sont montrés actifs chez la souris greffée par le mélanome B16 à des doses comprises entre 1 et 10 mg/kg par voie intrapéritonéale, ainsi que sur d'autres tumeurs liquides ou solides.

Les nouveaux produits ont des propriétés anti-tumorales et plus particulièrement une activité sur les tumeurs qui sont résistantes au Taxol® ou au Taxotère® . De telles tumeurs comprennent les tumeurs du colon qui ont une expression élevée du gène mdr 1 (gène de la multi-drug résistance). La multi-drug resistance est un terme habituel se rapportant à la résistance d'une tumeur à différents produits de structures et de mécanismes d'action différents. Les taxoïdes sont généralement connus pour être fortement reconnus par des tumeurs expérimentales telles que P388/DOX, une lignée cellulaire sélectionnée pour sa résistance à la doxorubicine (DOX) qui exprime mdr 1.

Les exemples suivants illustrent la présente invention.

### EXEMPLE 1

Une suspension de 120 mg de tert-butoxycarbonylamino-3 hydroxy-2 phényl-3 propionate-(2R,3S) de diacétoxy-4α,10β benzoyloxy-2α époxy-5β,20 dihydroxy-1β,19 oxo-9 trifluorométhanesulfonyloxy-7β taxène-11 yle-13α et de 143 mg de chlorure de sodium dans un mélange de 2 cm3 de tétrahydrofurane anhydre et 10 cm3 d'acétonitrile anhydre, maintenue sous atmosphère d'argon, est portée au reflux pendant 1 heure. Après refroidissement jusqu'à une température voisine de 20°C, le mélange réactionnel est filtré sur verre fritté, le résidu solide est rincé avec 10 cm3 d'acétate d'éthyle, et le filtrat est concentré sous pression réduite (2,7 kPa) à une température voisine de 30°C. On obtient ainsi 90 mg d'une meringue ivoire que l'on purifie par chromatographie préparative sur couche mince de silice [plaques préparatives Merck, Kieselgel 60F254 ; 20 x 20 cm ; épaisseur 0,5 mm ; dépôt en solution dans le dichlorométhane], en éluant par un mélange méthanol-dichlorométhane (5-95 en volumes). Après élution de la zone correspondant au produit recherché par un mélange méthanol-dichlorométhane (25-75 en volumes), filtration sur verre fritté, puis évaporation des solvants sous pression réduite (2,7 kPa) à une température voisine de 30°C, on obtient 65 mg de tert-butoxycarbonylamino-3 hydroxy-2 phényl-3 propionate-(2R,3S) de diacétoxy-4α,10β benzoyloxy-2α époxy-5β,20 hydroxy-1β oxo-9 nor-19 taxadiène-7,11 yle-13α sous forme de meringue blanche dont les caractéristiques sont les suivantes :
- spectre de R.M.N. ¹H (600 MHz ; CDCl₃ ; à une température de 333°K, δ en ppm ; constantes de couplage J en Hz) : 1,15 (s, 3H ; CH₃) ; 1,38 (s, 3H : CH₃) ; 1,42 (s, 9H : C(CH₃)₃) ; 1,72 (s, 3H : CH₃) ; 1,95 (s, 1H : OH) ; 2,00 et de 2,40 à 2,60 (respectivement dd et mt, J = 15 et 3, 1H chacun : CH₂ en 14) ; 2,23 (s, 3H : COCH₃) ; 2,27 (s, 3H : COCH₃) ; 2,52 (AB limite, J_{ab} = 17, 1H chacun : CH₂ en 6) ; 3,47 (d, J = 7,5 Hz, 1H : H en 3) ; 4,20 et 4,30 (2 d, J = 8, 1H chacun : CH₂ en 20) ; 4,25 (s large, 1H : OH en 2') ; 4,64 (mt, 1H : H en 2') ; 4,90 (s large, 1H : H en 5) ; 5,37 (d large, J = 10, 1H : H en 3') ; 5,58 (d, J = 7,5, 1H : H en 2) ; 5,67 (d, J = 10, 1H: CONH) ; 6,00 (dd, J = 6 et 3, 1H: H en 13) ; 6,21 (mt, 1H : H en 7) ; 6,35 (s, 1H : H en 10) ; 7,30 (t, J = 7,5, 1H: H en para de l'aromatique en 3') ; 7,38 (t, J = 7,5, 2H : H en méta de l'aromatique en 3') ; 7,47 (d, J = 7,5, 2H : H en ortho de l'aromatique en 3') ; 7,48 (t, J = 7,5, 2H : OCOC₆H₅ H en méta) ; 7,60 (t, J = 7,5, 1H : OCOC₆H₅ H en para) ; 8,12 (d, J = 7,5, 2H : OCOC₆H₅ H en ortho).

Le tert-butoxycarbonylamino-3 hydroxy-2 phényl-3 propionate-(2R,3S) de diacétoxy-4α,10β benzoyloxy-2α époxy-5β,20 dihydroxy-1β,19 oxo-9 trifluoro-méthanesulfonyloxy-7β taxène-11 yle-13α peut être préparé de la manière suivante :

Une solution de 147 mg de tert-butoxycarbonyl-3 (méthoxy-4 phényl)-2 phényl-4 oxazolidine-1,3 carboxylate-5 (2R,4S,5R) de diacétoxy-4α,10β benzoyloxy-2α époxy-5β,20 dihydroxy-1β,19 oxo-9 trifluorométhanesulfonyloxy-7β taxène-11 yle-13α dans 10 cm3 d'une solution 0,1N d'éthanol chlorhydrique est maintenue sous agitation à une température voisine de 20°C pendant 5 heures sous atmosphère d'argon. Le mélange réactionnel est alors dilué avec 50 cm3 d'acétate d'éthyle, 10 cm3 d'une solution aqueuse saturée en hydrogénocarbonate de sodium et 5 cm3 d'eau distillée. Après décantation, la phase organique est lavée avec 3 fois 15 cm3 d'eau distillée, séchée sur sulfate de magnésium, filtrée sur verre fritté puis concentrée à sec sous pression réduite (2,7 kPa) à une température voisine de 40°C. Le résidu obtenu est purifié par chromatographie préparative sur couche mince de silice [4 plaques préparatives Merck, Kieselgel 60F254 ; épaisseur 0,5 mm ; dépôt en solution dans le dichlorométhane], en éluant par un mélange méthanol-dichlorométhane (5-95 en volumes). Après élution de la zone correspondant au produit principal par un mélange méthanol-dichlorométhane (25-75 en volumes), filtration sur verre fritté, puis évaporation des solvants sous pression réduite (0,27 kPa) à une température voisine de 40°C, on obtient 120 mg de tert-butoxycarbonylamino-3 hydroxy-2 phényl-3 propionate-(2R,3S) de diacétoxy-4α,10β benzoyloxy-2α époxy-5β,20 dihydroxy-1β,19 oxo-9 trifluorométhanesulfonyloxy-7β taxène-11 yle-13α sous forme d'une meringue blanche dont les caractéristiques sont les suivantes :
- spectre de R.M.N. ¹H (300 MHz ; CDCl₃ ; à une température de 333°K, δ en ppm ; constantes de couplage J en Hz) : 1,24 (s, 3H : CH₃) ; 1,37 (s, 3H : CH₃) ; 1,37 (s, 9H : C(CH₃)₃) ; 1,87 (s, 1H : OH en 1) ; de 2,00 à 2,25 (mt, 2H : CH₂ en 14) ; 2,03 (s, 3H : CH₃) ; de 2,10 à 2,40 et 2,85 (2 mt, 1H chacun : CH₂ en 6) ; 2,25 (s, 3H : COCH₃) ; 2,45 (s, 3H : COCH₃) ; 2,45 (t, J = 6,5, 1H : OH en 19) ; 3,37 (d, J = 6, 1H : OH en 2') ; 3,95 (d, J = 7, 1H : H en 3) ; 4,40 (AB limite, J = 9, 2H : CH₂ en 20) ; 4,65 (mt, 1H : H en 2') ; 4,75 et 4,90 (2 dd, J = 13 et 6,5, 1H chacun : CH₂ en 19) ; 4,97 (d large, J = 10, 1H: H en 5) ; 5,27 (d large, J = 10, 1H : H en 3') ; 5,39 (d, J = 10, 1H : CONH) ; 5,48 (dd, J = 10 et 8, 1H : H en 7) ; 6,19 (t large, J = 9, 1H : H en 13) ; 6,65 (d, J = 7,1H : H en 2) ; 6,66 (s, 1H : H en 10); de 7,30 à 7,45 (mt, 5H : H aromatiques en 3') ; 7,52 (t, J = 7,5, 2H : OCOC₆H₅ H en méta) ; 7,65 (t, J = 7,5, 1H : OCOC₆H₅ H en para) ; 8,15 (d, J = 7,5, 2H : OCOC₆H₅ H en ortho).

A une solution de 280 mg de tert-butoxycarbonyl-3 (méthoxy-4 phényl)-2 phényl-4 oxazolidine-1,3 carboxylate-5-(2R,4S,5R) d'acétoxy-4α benzoyloxy-2α époxy-5β,20 trihydroxy-1β,10β,19 oxo-9 trifluorométhanesulfonyloxy-7β taxène-11 yle-13α dans 5 cm3 de pyridine anhydre, maintenue sous atmosphère d'argon, à une température voisine de 20°C, on ajoute successivement 15 mg de N,N'-diméthylamino-4-pyridine puis 0,07 cm3 d'anhydride acétique. Après 2 heures à une température voisine de 20°C, on ajoute 0,07 cm3 d'anhydride acétique. Deux heures plus tard on ajoute de nouveau 0,07 cm3 d'anhydride acétique. Après 2,5 heures à une température voisine de 20°C, le milieu réactionnel est dilué avec 25 cm3 d'acétate d'éthyle et 5 cm3 d'eau distillée. Après décantation, la phase organique est lavée avec 3 fois 5 cm3 d'eau distillée, séchée sur sulfate de magnésium, filtrée sur verre fritté puis concentrée à sec sous pression réduite (2,7 kPa) à une température voisine de 40°C. On obtient ainsi 287 mg d'une meringue ivoire que l'on purifie par chromatographie préparative sur couche mince de silice [4 plaques préparatives Merck, Kieselgel 60F254 ; épaisseur 0,5 mm ; dépôt en solution dans le dichlorométhane], en éluant par un mélange méthanol-dichlorométhane (5-95 en volumes). Après élution de la zone correspondant au produit principal par un mélange méthanol-dichlorométhane (25-75 en volumes), filtration sur verre fritté, puis évaporation des solvants sous pression réduite (0,27 kPa) à une température voisine de 40°C, on obtient 147 mg de tert-butoxycarbonyl-3 (méthoxy-4 phényl)-2 phényl-4 oxazolidine-1,3 carboxylate-5 (2R,4S,5R) de diacétoxy-4α,10β benzoyloxy-2α époxy-5β,20 dihydroxy-1β,19 oxo-9 trifluorométhanesulfonyloxy-7β taxène-11 yle-13α sous forme d'une meringue blanche.dont les caractéristiques sont les suivantes :
- spectre de R.M.N. ¹H (400 MHz ; CDCl₃ ; à une température de 333°K, δ en ppm ; constantes de couplage J en Hz) : 1,07 (s, 9H : C(CH₃)₃) ; 1,20 (s, 3H : CH₃) ; 1,34 (s, 3H : CH₃) ; 1,67 (s, 3H : CH₃) ; 1,82 (s, 1H : OH en 1) ; 1,91 (mf, 3H : COCH₃) ; 2,02 et 2,06 (2 dd, J = 15 et 8,5, 1H chacun : CH₂ en 14) ; 2,24 et 2,82 (2 mt, 1H chacun : CH₂ en 6) ; 2,24 (s, 3H : COCH₃) ; 2,40 (t, J = 7, 1H : OH en 19) ; 3,84 (s, 3H : ArOCH₃) ; 3,85 (d, J = 7, 1H : H en 3) ; 4,30 et 4,37 (2 d, J = 8, 1H chacun : CH₂ en 20) ; 4,58 (d, J = 5, 1H : H en 2') ; 4,71 et 4,83 (2 dd, J = 13 et 7, 1H chacun : CH₂ en 19) ; 4,90 (d large, J = 10, 1H : H en 5) ; 5,45 (dd, J = 10,5 et 7, 1H : H en 7) ; 5,45 (mf, 1H : H en 3') ; 6,07 (t large, J = 9, 1H : H en 13) ; 6,40 (mf, 1H : H en 5') ; 6,54 (s, 1H : H en 10) ; 6,61 (d, J = 7, 1H : H en 2) ; 6,94 (d, J = 8, 2H : H aromatiques en ortho du OCH₃) ; de 7,30 à 7,45 (mt, 7H : H aromatiques en 3' et H aromatiques en méta du OCH₃) ; 7,51 (t, J = 7,5, 2H : OCOC₆H₅ H en méta) ; 7,63 (t, J = 7,5, 1H : OCOC₆H₅ H en para) ; 8,07 (d, J = 7,5, 2H : OCOC₆H₅ H en ortho).

A une suspension de 3,3 g de tert-butoxycarbonyl-3 (méthoxy-4 phényl)-2 phényl-4 oxazolidine-1,3 carboxylate-5-(2R,4S,5R) d'acétoxy-4α benzoyloxy-2α époxy-5β,20 tétrahydroxy-1β,7β,10β,19 oxo-9 taxène-11 yle-13α et de 1,5 g de tamis moléculaire activé 4Å dans 60 cm3 de dichlorométhane anhydre et 0,97 cm3 de pyridine anhydre, maintenue sous atmosphère d'argon, à une température voisine de -35°C, on ajoute goutte à goutte 1,26 cm3 d'anhydride trifluorométhanesulfonique. Après une heure à une température voisine de 0°C, le mélange réactionnel est refroidi à une température voisine de -10°C, dilué avec 20 cm3 d'eau distillée et filtré sur verre fritté garni de célite. Après rinçage du verre fritté avec 20 cm3 de dichlorométhane, et décantation du filtrat, la phase organique est lavée avec 2 fois 25 cm3 d'eau distillée, séchée sur sulfate de magnésium, filtrée sur verre fritté puis concentrée à sec sous pression réduite (2,7 kPa) à une température voisine de 40°C. On obtient ainsi 3,2 g d'une meringue jaune que l'on purifie par chromatographie à pression atmosphérique sur silice (0,043-0,060 mm) contenu dans une colonne de 2,5 cm de diamètre et 30 cm de hauteur en éluant avec 1 litre de dichlorométhane puis avec un mélange méthanol-dichlorométhane (1-99 en volumes) en recueillant des fractions de 15 cm3. Les fractions ne contenant que le produit cherché sont réunies et concentrées à sec sous pression réduite (2,7 kPa) à 40°C pendant 2 heures. On obtient ainsi 1,35 g de tert-butoxycarbonyl-3 (méthoxy-4 phényl)-2 phényl-4 oxazolidine-1,3 carboxylate-5-(2R,4S,SR) d'acétoxy-4α benzoyloxy-2α époxy-5β,20 trihydroxy-1β,10β,19 oxo-9 trifluorométhanesulfonyloxy-7β taxène-11 yle-13α sous forme d'une meringue blanche dont les caractéristiques sont les suivantes :
- spectre de R.M.N. ¹H (300 MHz ; CDCL₃ ; à une température de 333°K, δ en ppm ; constantes de couplage J en Hz) : 1,08 (s, 9H : C(CH₃)₃) ; 1,23 (s, 6H : CH₃) ; 1,56 (s, 3H : CH₃) ; 1,78 (s, 1H : OH en 1); 1,93 (mf, 3H : COCH₃) ; de 1,85 à 2,15 et 2,76 (2 mts, 1H chacun : CH₂ en 6) ; de 1,85 à 2,15 et 2,20 (respectivement mt et dd, J = 16 et 9 Hz, 1H chacun : CH₂ en 14) ; 2,48 (t, J = 6 Hz, 1H : OH en 19) ; 3,82 (s, 3H : ArOCH₃) ; 3,90 (d, J = 7,1H : H en 3) ; 3,96 (s large, 1H : OH en 10) ; 4,30 et 4,45 (2 d, J = 9, 1H chacun : CH₂ en 20) ; 4,58 (d, J = 5, 1H : H en 2') ; 4,75 et 4,86 (2 dd, J = 13 et 6, 1H chacun : CH₂ en 19) ; 4,91 (d large, J = 10, 1H: H en 5) ; 5,30 (s large, 1H: H en 10) ; 5,40 (d, J = 11 et 8, 1H: H en 7) ; 5,45 (mf, 1H: H en 3') ; 6,15 (t large, J = 9, 1H : H en 13) ; 6,40 (mf, 1H : H en 5') ; 6,58 (d, J = 7, 1H : H en 2) ; 6,95 (d, J = 8, 2H : H aromatiques en ortho du OCH₃) ; de 7,25 à 7,45 (mt, 7H : H aromatiques en 3' et H aromatiques en méta du OCH₃) ; 7,50 (t, J = 7,5, 2H : OCOC₆H₅ H en méta) ; 7,66 (t, J = 7,5, 1H : OCOC₆H₅ H en para) ; 8,06 (d, J = 7,5, 2H : OCOC₆H₅ H en ortho).

A une suspension de 4,4 g de tert-butoxycarbonyl-3 (méthoxy-4 phényl)-2 phényl-4 oxazolidine-1,3 carboxylate-5-(2R,4S,5R) d'acétoxy-4α benzoyloxy-2α époxy-5β,20 hydroxy-1β oxo-9 tris-(trichloro-2,2,2 éthoxycarbonyloxy)-7β,10β,19 taxène-11 yle-13α et 4,88 g de zinc en poudre dans 40 cm3 d'acétate d'éthyle, maintenue sous atmosphère d'argon, à une température voisine de 20°C, on ajoute goutte à goutte 1,03 cm3 d'acide acétique. Après 2 heures à une température voisine de 20°C, le mélange réactionnel est porté à une température voisine de 40°C, et sont ajoutés 4,88 g de zinc en poudre et 1,03 cm3 d'acide acétique. Après 1 heure à une température voisine de 40°C, le mélange réactionnel est refroidi jusqu'à une température voisine de 20°C, dilué avec 200 cm3 d'acétate d'éthyle et 100 cm3 d'une solution aqueuse saturée en hydrogénocarbonate de sodium, filtré sur verre fritté garni de célite. Après rinçage du verre fritté avec 75 cm3 d'acétate d'éthyle, et décantation du filtrat, la phase organique est lavée avec 4 fois 100 cm3 d'eau distillée, séchée sur sulfate de magnésium, filtrée sur verre fritté puis concentrée à sec sous pression réduite (2,7 kPa) à une température voisine de 40°C. On obtient ainsi 3,3 g de tert-butoxycarbonyl-3 (méthoxy-4 phényl)-2 phényl-4 oxazolidine-1,3 carboxylate-5-(2R,4S,5R) d'acétoxy-4α benzoyloxy-2α époxy-5β,20 tétrahydroxy-1β,7β-10β,19 oxo-9 taxéne-11 yle-13α sous forme d'une meringue blanche dont les caractéristiques sont les suivantes :
- spectre de R.M.N. ¹H (300 MHz ; CDCl₃ ; à une température de 333°K ; δ en ppm ; constantes de couplage J en Hz) : 1,07 (s, 9H : C(CH₃)₃) ; 1,22 (s, 3H : CH₃) ; 1,24 (s, 3H : CH₃) ; 1,52 (s, 3H : CH₃) ; 1,67 (s, 1H : OH en 1) ; 1,85 (mf, 3H : COCH₃) ; de 1,85 à 2,10 et 2,68 (2 mt, 1H chacun : CH₂ 6) ; de 1,85 à 2,10 et 2,14 (respectivement mt et dd, J = 16 et 9, 1H chacun : CH₂ en 14) ; 2,68 (d, J = 10, 1H: OH en 7) ; 3,21 (dd, J = 8 et 5, 1H : OH en 19) ; 3,80 (d, J = 7, 1H : H en 3) ; 3,83 (s, 3H : ArOCH₃) ; 4,02 (s large, 1H : OH en 10) ; 4,02 et 4,30 (2 d, J = 9, 1H chacun : CH₂ en 20) ; 4,28 (mt, 1H : H en 7) ; 4,57 (d, J = 5, 1H : H en 2') ; 4,71 et 4,80 (2 dd, respectivement J = 13 et 5 et J = 13 et 8, 1H chacun ; CH₂ en 19) ; 4,94 (d large, J = 10, 1H : H en 5) ; 5,20 (s large, 1H : H en 10) ; 5,42 (mf, 1H : H en 3') ; 5,80 (d, J = 7, 1H : H en 2) ; 6,14 (t large, J = 9, 1H : H en 13) ; 6,39 (mf, 1H : H en 5') ; 6,94 (d, J = 8, 2H : H aromatiques en ortho du OCH₃) ; de 7,25 à 7,45 (mt, 7H : H aromatiques en 3' et H aromatiques en méta du OCH₃) ; 7,50 (t, J = 7,5, 2H : OCOC₆H₅ H en méta) ; 7,63 (t, J = 7,5, 1H : OCOC₆H₅ H en para) ; 8,03 (d, J = 7,5, 2H : OCOC₆H₅ H en ortho).

A une solution de 10,86 g d'acétoxy-4α benzoyloxy-2α époxy-5β,20 hydroxy-1β oxo-9 tris-(trichloro-2,2,2 éthoxycarbonyloxy)-7β,10β,19 taxène-11 et 5,19 g d'acide (2R,4S,5R)-tert-butoxycarbonyl-3 (méthoxy-4 phényl)-2 phényl-4 oxazolidine-1,3 carboxylique-5-(2R,4S,5R) dans 150 cm3 d'acétate d'éthyle, maintenue sous atmosphère d'argon, à une température voisine de 20°C, on ajoute successivement 3,3 g de dicyclohexylcarbodiimide puis 0,366 g de N,N'-diméthylamino-4 pyridine, Après 16 heures, à une température voisine de 20°C, la suspension obtenue est filtrée sur verre fritté. Après rinçage du verre fritté avec 50 cm3 d'acétate d'éthyle, le filtrat est dilué avec 100 cm3 d'acétate d'éthyle, lavé par 100 cm3 d'une solution aqueuse saturée en hydrogénocarbonate de sodium, puis par 4 fois 100 cm3 d'eau distillée, séché sur sulfate de magnésium, filtré sur verre fritté puis concentré à sec sous pression réduite (2,7 kPa) à une température voisine de 40°C. On obtient ainsi 15,7 g de tert-butoxycarbonyl-3 (méthoxy-4 phényl)-2 phényl-4 oxazolidine-1,3 carboxylate-5-(2R,4S,5R) d'acétoxy-4α benzoyloxy-2α époxy-5β,20 hydroxy-1β oxo-9 tris-(trichloro-2,2,2 éthoxycarbonyloxy)-7β,10β,19 taxène-11 yle-13α sous forme d'une meringue blanche dont les caractéristiques sont les suivantes:
- spectre de R.M.N. ¹H (400 MHz ; CDCl₃ ; à une température de 333°K, δ en ppm ; constantes de couplage J en Hz) : 1,06 (s, 9H : C(CH₃)₃) ; 1,27 (s, 3H : CH₃) ; 1,33 (s, 3H : CH₃) ; 1,68 (s, 3H : CH₃) ; 1,86 et 2,65 (2 mt, 1H chacun : CH₂ en 6) ; 1,92 (s, 3H : COCH₃) ; 2,07 et 2,26 (2 dd, J = 15,5 et 9, 1H chacun : CH₂ en 14) ; 3,84 (s, 3H : ArOCH₃) ; 3,94 (d, J = 7,1H : H en 3) ; 4,17 et 4,34 (2 d, J = 9,1H chacun : CH₂ en 20) ; 4,58 (d, J = 5, 1H : H en 2') ; 4,61 et 4,93 (2 d, J = 12, 1H chacun : COOCH₂CCl₃) ; 4,75 et 4,81 (2 d, J = 12, 1H chacun : COOCH₂CCl₃) ; 4,75 et 4,99 (2 d, J = 12, 1H chacun : COOCH₂CCl₃) ; 4,92 (d large, J = 10, 1H : H en 5) ; 5,43 (d, J = 5, 1H : H en 3') ; 5,43 (AB limite, J = 10,5, 2H : CH₂ en 19) ; 5,58 (dd, J = 10,5 et 7,1H : H en 7) ; 6,13 (t large, J = 9, 1H : H en 13) ; 6,20 (s, 1H : H en 10) ; 6,38 (s, 1H : H en 5') ; 6,39 (d, J = 7, 1H : H en 2) ; 6,94 (d, J = 8, 2H : H aromatiques en ortho du OCH₃) ; de 7,25 à 7,45 (mt, 7H : H aromatiques en 3' et H aromatiques en méta du OCH₃) ; 7,50 (t, J = 7,5, 2H : OCOC₆H₅ H en méta) ; 7,62 (t, J = 7,5, 1H : OCOC₆H₅ H en para) ; 8,04 (d, J = 7,5, 2H : OCOC₆H₅ H en ortho).

### EXEMPLE 2

Une suspension de 187,3 mg de tert-butoxycarbonyl-3 diméthyl-2,2 phényl-4 oxazolidinecarboxylate-5-(4S,5R) d'acétoxy-4α benzoyloxy-2α époxy-5β,20 trihydroxy-1β,10β,19 oxo-9 trifluorométhanesulfonyloxy-7β taxène-11 yle-13α, 112 mg de tamis moléculaire 4Å activé et de 171 mg de chlorure de sodium dans un mélange de 1,7 cm3 de tétrahydrofurane anhydre et 6,8 cm3 d'acétonitrile anhydre, maintenue sous atmosphère d'argon, est portée au reflux pendant 1,5 heure. Après refroidissement jusqu'à une température voisine de 20°C, le mélange réactionnel est filtré sur verre fritté, le résidu solide est rincé avec 10 cm3 d'acétonitrile, et le filtrat est concentré sous pression réduite (2,7 kPa) à une température voisine de 40°C. Le résidu obtenu est purifié par chromatographie préparative sur couche mince de silice [4 plaques préparatives Merck, Kieselgel 60F254 ; 20 x 20 cm ; épaisseur 0,5 mm ; dépôt en solution dans le dichlorométhane], en éluant par un mélange méthanol-dichlorométhane (3-97 en volumes). Après élution de la zone correspondant au produit recherché par un mélange méthanol-dichlorométhane (15-85 en volumes), filtration sur verre fritté, puis évaporation des solvants sous pression réduite (2,7 kPa) à une température voisine de 40°C, on obtient 55 mg de tert-butoxycarbonyl-3 diméthyl-2,2 phényl-4 oxazolidinecarboxylate-5-(4S,5R) d'acétoxy-4α benzoyloxy-2α époxy-5β,20 dihydroxy-1β,10β oxo-9 nor-19 taxadiène-7,11 yle-13α sous forme de meringue blanche dont les caractéristiques sont les suivantes:
- spectre de R.M.N. ¹H (400 MHz ; CDCl₃ ; δ en ppm ; constantes de couplage J en Hz) : 1,13 (mf, 9H : C(CH₃)₃) ; 1,20 (s, 3H : CH₃) ; 1,31 (s, 3H :CH₃) ; 1,70 (s, 3H : CH₃) ; 1,73 (s, 3H : CH₃) ; 1,83 (s, 6H : CH₃ et COCH₃) ; 1,90 et 2,43 (2 dd, respectivement J = 16 et 6 et J = 16 et 10,1 1 H chacun: CH₂ en 14) ; 1,95 (s, 1H: OH en 1) ; 2,45 et 2,60 (2 d larges, J = 18, 1H chacun : CH₂ en 6) ; 3,40 (d large, J = 8, 1H : H en 3) ; 3,78 (mf, 1H : OH en 10) ; 4,10 et 4,26 (2 d, J = 8, 2H : CH₂ en 20) ; 4,56 (d, J = 6, 1H : H en 2') ; 4,88 (s large, 1H : H en 5) ; 5,19 (mf, 1H : H en 3') ; 5,19 (s, 1H : H en 10) ; 5,50 (d, J = 8, 1H ; H en 2) ; 6,03 (dd, J = 10 et 6, 1H : H en 13) ; 6,27 (mt, 1H : H en 7) ; 7,35 (mt, 5H : H aromatiques en 3') ; 7,48 (t, J = 7,5, 2H : OCOC₆H₅ H en méta) ; 7,62 (t, J = 7,5, 1H : OCOC₆H₅ H en para) ; 8,08 (d, J = 7,5, 2H : OCOC₆H₅ H en ortho).

Le tert-butoxycarbonyl-3 diméthyl-2,2 phényl-4 oxazolidinecarboxylate-5-(4S,5R) d'acétoxy-4α benzoyloxy-2α époxy-5β,20 dihydroxy-1β,10β oxo-9 nor-19 taxadiène-7,11 yle-13α traité par l'acide formique à 20°C fournit l'amino-3 hydroxy-2 phényl-3 propionate-(2R,3S) d'acétoxy-4α-benzoyloxy-2α époxy-5β,20 dihydroxy-1β,10β oxo-9 nor-19 taxadiène-7,11 yle-13α qui réagit avec le dicarbonate de di-tert-butyle en présence d'hydrogénocarbonate de sodium dans le dichlorométhane à 20°C fournit le tert-butoxycarbonylamino-3 hydroxy-2 phényl-3 propionate-(2R,3S) d'acétoxy-4α-benzoyloxy-2α époxy-5β,20 dihydroxy-1β,10β oxo-9 nor-19 taxadiène-7,11 yle-13α.

Le tert-butoxycarbonyl-3 diméthyl-2,2 phényl-4 oxazolidinecarboxylate-5-(4S,5R) d'acétoxy-4α benzoyloxy-2α époxy-5β,20 trihydroxy-1β,10β,19 oxo-9 trifluorométhanesulfonyloxy-7β taxène-11 yle-13α peut être préparé de la manière suivante :

A une suspension de 316 mg de tert-butoxycarbonyl-3 diméthyl-2,2 phényl-4 oxazolidinecarboxylate-5-(4S,5R) d'acétoxy-4α benzoyloxy-2α époxy-5β,20 tétrahydroxy-1β,7β,10β,19 oxo-9 taxène-11 yle-13α et de 25 mg de tamis moléculaire activé 4 Å dans 3 cm3 de dichlorométhane anhydre et 0,12 cm3 de pyridine anhydre, maintenue sous atmosphère d'argon, à une température voisine de -35°C, on ajoute goutte à goutte 0,155 cm3 d'anhydride trifluorométhanesulfonique. Après 50 minutes à une température voisine de 0°C, le mélange réactionnel est refroidi à une température voisine de -15°C, dilué avec 5 cm3 d'eau distillée, 3 cm3 d'une solution aqueuse saturée en chlorure de sodium et 10 cm3 de dichlorométhane. Après décantation, la phase aqueuse est extraite avec 5 cm3 de dichlorométhane. Les phases organiques rassemblées sont lavées avec 5 cm3 d'eau distillée, séchée sur sulfate de magnésium, filtrée sur verre fritté puis concentrée à sec sous pression réduite (2,7 kPa) à une température voisine de 40°C. On obtient ainsi 373,6 mg d'un produit brut que l'on purifie par chromatographie préparative sur couche mince de silice [10 plaques préparatives Merck Kieselgel 60F254 ; 20 x 20 cm ; épaisseur 1 mm ; dépôt en solution dans le dichlorométhane], en éluant par un mélange méthanol-dichlorométhane (5-95 en volumes). Après élution de la zone correspondant au produit recherché par un mélange méthanol-dichlorométhane (15-85 en volumes), filtration sur verre fritté, puis évaporation des solvants sous pression réduite (2,7 kPa) à une température voisine de 40°C, on obtient 187,3 mg de tert-butoxycarbonyl-3 diméthyl-2,2 phényl-4 oxazolidinecarboxylate-5-(4S,5R) d'acétoxy-4α benzoyloxy-2α époxy-5β,20 trihydroxy-1β,10β,19 oxo-9 trifluorométhanesulfonyloxy-7β taxène-11 yle-13α sous forme de meringue blanche dont les caractéristiques sont les suivantes:
- spectre de R.M.N. ¹H (400 MHz ; CDCl₃ ; à une température de 333°K ; δ en ppm ; constantes de couplage J en Hz) : 1,23 (s, 9H : C(CH₃)₃) ; 1,29 (s, 6H : CH₃) ; 1,75 (s, 1H : OH en 1) ; 1,79 (s, 3H : CH₃) ; 1,85 (s, 3H : CH₃) ; 2,01 (s, 3H : COCH₃) ; 2,03 (s, 3H : CH₃) ; 2,12 et 2,23 (2 dd, J = 16 et 9, 1 H chacun: CH₂ en 14) ; 2,13 et 2,81 (2 mts. 1H chacun : CH₂ en 6) ; 2,39 (t, J = 7, 1H : OH en 19) ; 3,91 (s large, 1H : OH en 10) ; 4,03 (d, J = 7, 1H : H en 3) ; 4,37 (AB limite, J = 9, 2H : CH₂ en 20) ; 4,47 (d, J = 7, 1H : H en 2') ; 4,78 et 4,89 (2 dd, J = 12 et 7, 1H chacun : CH₂ en 19) ; 4,94 (d large, J = 10, 1H: H en 5) ; 5,17 (d, J = 7, 1H: H en 3') ; 5,43 (s large, 1H: H en 10) ; 5,50 (dd, J = 11 et 8, 1H : H en 7) ; 6,30 (t large, J = 9, 1H : H en 13) ; 6,58 (d, J = 7,1H : H en 2) ; 7,38 (mt, 5H : H aromatiques en 3') ; 7,50 (t, J = 7,5, 2H : OCOC₆H₅ H en méta) ; 7,63 (t, J = 7,5, 1H : OCOC₆H₅ H en para) ; 8,07 (d, J = 7,5, 2H : OCOC₆H₅ H en ortho).

Le tert-butoxycarbonyl-3 diméthyl-2,2 phényl-4 oxazolidinecarboxylate-5-(4S,5R) d'acétoxy-4α benzoyloxy-2α époxy-5β,20 tétrahydroxy-1β,7β,10β,19 oxo-9 taxène-11 yle-13α peut être préparé de la manière suivante :

A une suspension de 500 mg de tert-butoxycarbonyl-3 diméthyl-2,2 phényl-4 oxazolidinecarboxylate-5-(4S,5R) d'acétoxy-4α benzoyloxy-2α époxy-5β,20 hydroxy-1β oxo-9 tris-(trichloro-2,2,2 éthoxycarbonyloxy)-7β,10β,19 taxène-11 yle-13α et 585 mg de zinc en poudre dans 1,5 cm3 d'acétate d'éthyle, maintenue sous atmosphère d'argon, à une température voisine de 20°C, on ajoute goutte à goutte 0,117 cm3 d'acide acétique. Après 20 minutes à une température voisine de 20°C, le mélange réactionnel est dilué avec 15 cm3 d'acétate d'éthyle, 10 cm3 d'une solution aqueuse saturée en hydrogénocarbonate de sodium et 5 cm3 d'eau distillée, filtré sur verre fritté garni de célite. Après rinçage du verre fritté avec 5 cm3 d'acétate d'éthyle, et décantation du filtrat, la phase aqueuse est extraite avec 5 cm3 d'acétate d'éthyle. Les phases organiques rassemblées sont lavées avec 5 cm3 d'eau distillée, séchées sur sulfate de magnésium, filtrées sur verre fritté puis concentrées à sec sous pression réduite (2,7 kPa) à une température voisine de 40°C. On obtient ainsi 316 mg de tert-butoxycarbonyl-3 diméthyl-2,2 phényl-4 oxazolidinecarboxylate-5-(4S,5R) d'acétoxy-4α benzoyloxy-2α époxy-5β,20 tétrahydroxy-1β,7β,10β,19 oxo-9 taxène-11 yle-13α sous forme d'une meringue blanche dont les caractéristiques sont les suivantes :
- spectre de R.M.N. ¹H (400 MHz ; CDCl3 ; δ en ppm ; constantes de couplage J en Hz) : 1,10 (mf, 9H : C(CH₃)₃) ; 1,24 (s, 3H : CH₃) ; 1,27 (s, 3H : CH₃) ; 1,67 (mf, 1H : OH en 1) ; 1,77 (s, 3H : CH₃) ; 1,82 (s, 3H : CH₃) ; 1,88 (s, 3H : CH₃) ; 2,01 (s, 3H : COCH₃) ; 2,05 et 2,70 (2 mt, 1H chacun : CH₂ en 6) ; 2,12 (AB limite, J = 15 et 9, 2H : CH₂ en 14) ; 3,20 (mf, 1H : OH) ; 3,96 (d, J = 7,1H : H en 3) ; 4,07 et 4,32 (2 d, J = 8,5,1H chacun : CH₂ en 20) ; 4,07 (mf, 1H : OH) ; 4,39 (mt,1H : H en 7) ; 4,46 (d, J = 7, 1H: H en 2') ; 4,74 et 4,81 (respctivement d large et dd, J = 11 et J = 11 et 4, 1H chacun : CH₂ en 19) ; 4,98 (d large, J = 10, 1H: H en 5) ; 5,08 (mf, 1H : H en 3') ; 5,28 (s, 1H : H en 10) ; 5,82 (d, J = 7,1H : H en 2) ; 6,27 (t large, J = 9, 1H : H en 13) ; 7,35 (mt, 5H : H aromatiques en 3') ; 7,50 (t, J = 7,5, 2H : OCOC₆H₅ H en méta) ; 7,63 (t, J = 7,5, 1H : OCOC₆H₅ H en para) ; 8,04 (d, J = 7,5, 2H : OCOC₆H₅ H en ortho).

Le tert-butoxycarbonyl-3 diméthyl-2,2 phényl-4 oxazolidinecarboxylate-5-(4S,5R) d'acétoxy-4α benzoyloxy-2α époxy-5β,20 hydroxy-1β oxo-9 tri-((trichloro-2,2,2 éthoxy)carbonyloxy)-7β,10β,19 taxène-11 yle-13α peut être préparé de la manière suivante:

A une solution de 10,9 g d'acétoxy-4α benzoyloxy-2α époxy-5β,20 hydroxy-1β oxo-9 tris-(trichloro-2,2,2 éthoxycarbonyloxy)-7β,10β,19 taxéne-11 et 4,19 g d'acide tert-butoxycarbonyl-3 diméthyl-2,2 phényl-4 oxazolidinecarboxylique-5-(4S,5R) dans 50 cm3 de toluène anhydre, maintenue sous atmosphère d'argon, à une température voisine de 20°C, on ajoute successivement 3,31 g de dicyclohexylcarbodiimide puis 0,370 g de N,N'-diméthylamino-4 pyridine. Après 20 heures, à une température voisine de 20°C, le mélange réactionnel brut est purifié directement par dépôt sur une colonne de chromatographie à pression atmosphérique contenant 400 g de silice (0,063-0,2 mm) contenus dans une colonne de 5 cm de diamètre, en éluant avec un mélange méthanol-dichlorométhane (0,5-99,5 puis 1-99 en volumes) en recueillant des fractions de 50 cm3. Les fractions ne contenant que le produit cherché sont réunies et concentrées à sec sous pression réduite (2,7 kPa) à 40°C pendant 2 heures. On obtient ainsi 11,0 g de tert-butoxycarbonyl-3 diméthyl-2,2 phényl-4 oxazolidinecarboxylate-5-(4S,5R) d'acétoxy-4α benzoyloxy-2α époxy-5β,20 hydroxy-1β oxo-9 tris-(trichloro-2,2,2 éthoxycarbonyloxy)-7β,10β,19 taxène-11 yle-13α sous forme d'une meringue blanche dont les caractéristiques sont les suivantes :
- spectre de R.M.N. ¹H (400 MHz ; CDCl3 ; à une température de 333°K ; δ en ppm ; constantes de couplage J en Hz) : 1,22 (mf, 9H : C(CH₃)₃) ; 1,31 (s, 3H : CH₃) ; 1,37 (s, 3H : CH₃) ; 1,77 (s, 3H : CH₃) ; 1,85 (s, 3H : CH₃) ; 1,85 et 2,69 (2 mt, 1H chacun : CH₂ en 6) ; 2,01 (s, 3H : CH₃) ; 2,09 (s, 3H : COCH₃) ; 2,14 et 2,25 (2 dd, J = 15 et 9, 1H chacun : CH₂ en 14) ; 4,03 (d, J = 7, 1H: H en 3) ; 4,20 et 4,37 (2 d, J = 8,5, 1H chacun : CH₂ en 20) ; 4,49 (d, J = 7, 1H: H en 2') ; 4,64 et 4,94 (2 d, J = 12, 1H chacun : COOCH₂CCl₃) ; 4,78 et 4,82 (2 d, J = 12, 1H chacun : COOCH₂CCl₃) ; 4,78 et 4,97 (2 d, J =12, 1H chacun : COOCH₂CCl₃) ; 4,96 (d large, J = 10, 1H : H en 5) ; 5,16 (d, J = 7, 1H: H en 3') ; 5,44 et 5,49 (2 d, J = 10, 2H : CH₂ en 19) ; 5,66 (dd, J = 11 et 8, 1H : H en 7) ; 6,29 (t large, J = 9, 1H: H en 13) ; 6,29 (s, 1H : H en 10) ; 6,44 (d, J = 7, 1H : H en 2) ; de 7,30 à 7,45 (mt, 5H : H aromatiques en 3') ; 7,50 (t, J = 7,5, 2H : OCOC₆H₅ H en méta) ; 7,63 (t, J = 7,5, 1H: OCOC₆H₅ H en para) ; 8,07 (d, J = 7,5, 2H : OCOC₆H₅ H en ortho).

Les nouveaux produits de formule générale (I) dans laquelle Z représente un radical de formule générale (II) manifestent une activité inhibitrice significative de la prolifération cellulaire anormale et possèdent des propriétés thérapeutiques permettant le traitement de malades ayant des conditions pathologiques associées à une prolifération cellulaire anormale. Les conditions pathologiques incluent la prolifération cellulaire anormale de cellules malignes ou non malignes de divers tissus et/ou organes, comprenant, de manière non limitative, les tissus musculaires, osseux ou conjonctifs, la peau, le cerveau, les poumons, les organes sexuels, les systèmes lymphatiques ou rénaux, les cellules mammaires ou sanguines, le foie, l'appareil digestif, le pancréas et les glandes thyroïdes ou adrénales. Ces conditions pathologiques peuvent inclure également le psoriasis, les tumeurs solides, les cancers de l'ovaire, du sein, du cerveau, de la prostate, du colon, de l'estomac, du rein ou des testicules, le sarcome de Kaposi, le cholangiocarcinome, le choriocarcinome, le neuroblastome, la tumeur de Wilms, la maladie de Hodgkin, les mélanomes, les myélomes multiples, les leucémies lymphocytaires chroniques, les lymphomes granulocytaires aigus ou chroniques. Les nouveaux produits selon l'invention sont particulièrement utiles pour le traitement du cancer de l'ovaire. Les produits selon l'invention peuvent être utilisés pour prévenir ou retarder l'apparition ou la réapparition des conditions pathologiques ou pour traiter ces conditions pathologiques.

Les produits selon l'invention peuvent être administrés à un malade selon différentes formes adaptées à la voie d'administration choisie qui, de préférence, est la voie parentérale. L'administration par voie parentérale comprend les administrations intraveineuse, intrapéritonéale, intramusculaire ou sous-cutanée. Plus particulièrement préférée est l'administration intrapéritonéale ou intraveineuse.

La présente invention comprend également les compositions pharmaceutiques qui contiennent au moins un produit de formule générale (I) en une quantité suffisante adaptée à l'emploi en thérapeutique humaine ou vétérinaire. Les compositions peuvent être préparées selon les méthodes habituelles en utilisant un ou plusieurs adjuvants, supports ou excipients pharmaceutiquement acceptables. Les supports convenables incluent les diluants, les milieux aqueux stériles et divers solvants non toxiques. De préférence les compositions se présentent sous forme de solutions ou de suspensions aqueuses, de solutions injectables qui peuvent contenir des agents émusifiants, des colorants, des préservatifs ou des stabilisants. Cependant, les compositions peuvent aussi se présenter sous forme de comprimés, de pilules, de poudres ou de granulés administrables par voie orale.

Le choix des adjuvants ou excipients peut être déterminé par la solubilité et les propriétés chimiques du produit, le mode particulier d'administration et les bonnes pratiques pharmaceutiques.

Pour l'administration parentérale, on utilise des solutions ou des suspensions stériles aqueuses ou non aqueuses. Pour la préparation de solutions ou de suspensions non aqueuses peuvent être utilisés des huiles végétales naturelles telle que l'huile d'olive, l'huile de sésame ou l'huile de paraffine ou les esters organiques injectables tel que l'oléate d'éthyle. Les solutions stériles aqueuses peuvent être constituées d'une solution d'un sel pharmaceutiquement acceptable en solution dans de l'eau. Les solutions aqueuses conviennent pour l'administration intraveineuse dans la mesure où le pH est convenablement ajusté et où l'isotonicité est réalisée, par exemple, par une quantité suffisante de chlorure de sodium ou de glucose. La stérilisation peut être réalisée par chauffage ou par tout autre moyen qui n'altère pas la composition.

Il est bien entendu que tous les produits entrant dans les compositions selon l'invention doivent être purs et non toxiques pour les quantités utilisées.

Les compositions peuvent contenir au moins 0,01 % de produit thérapeutiquement actif. La quantité de produit actif dans une composition est telle qu'une posologie convenable puisse être prescrite. De préférence, les compositions sont préparées de telle façon qu'une dose unitaire contienne de 0,01 à 1000 mg environ de produit actif pour l'administration par voie parentérale.

Le traitement thérapeutique peut être effectué concuremment avec d'autres traitements thérapeutiques incluant des médicaments antinéoplastiques , des anticorps monoclonaux, des thérapies immunologiques ou des radiothérapies ou des modificateurs des réponses biologiques. Les modificateurs des réponses incluent, de manière non limitative, les lymphokines et les cytokines telles que les interleukines, les interférons (α, β ou δ) et le TNF. D'autres agents chimiothérapeutiques utiles dans le traitement des désordres dus à la prolifération anormale des cellules incluent, de manière non limitative, les agents alkylants tels que les moutardes à l'azote comme la mechloretamine, le cyclophosphamide, le melphalan et le chlorambucil, des sulfonates d'alkyle comme le busulfan, les nitrosourées comme la carmustine, la lomustine, la sémustine et la streptozocine, les triazènes comme la dacarbazine, les antimétabolites comme les analogues de l'acide folique tel que le méthotrexate, les analogues de pyrimidine comme le fluorouracil et la cytarabine, des analogues de purines comme la mercaptopurine et la thioguanine, des produits naturels tels que les alcaloïdes de vinca comme la vinblastine, la vincristine et la vendésine, des épipodophyllotoxines comme l'étoposide et le teniposide, des antibiotiques comme la dactinomycine, la daunorubicine, la doxorubicine, la bléomycine, la plicamycine et la mitomycine, des enzymes comme la L-asparaginase, des agents divers comme les complexes de coordination du platine tel que le cisplatine, les urées substituées telles que l'hydroxyurée, les dérivés de méthylhydrazine comme la procarbazine, les suppresseurs adrénocoticoïdes comme le mitotane et l'aminoglutéthymide, les hormones et les antagonistes comme les adrénocorticostéroïdes comme la prednisone, les progestines comme le caproate d'hydroxyprogestérone, l'acétate de méthoxyprogestérone et l'acétate de megestrol, les oestrogènes comme le diéthylstilbestrol et l'éthynylestradiol, les antioestrogènes comme le tamoxifène, les androgènes comme le propionate de testostérone et la fluoxymesterone.

Les doses utilisées pour mettre en oeuvre les méthodes selon l'invention sont celles qui permettent un traitement prophylactique ou un maximum de réponse thérapeutique. Les doses varient selon la forme d'administration, le produit particulier sélectionné et les caractéristiques propres du sujet à traiter. En général, les doses sont celles qui sont thérapeutiquement efficaces pour le traitement des désordres dus à une prolifération cellulaire anormale. Les produits selon l'invention peuvent être administrés aussi souvent que nécessaire pour obtenir l'effet thérapeutique désiré. Certains malades peuvent répondre rapidement à des doses relativement fortes ou faibles puis avoir besoin de doses d'entretien faibles ou nulles. Généralement, de faibles doses seront utilisées au début du traitement et, si nécessaire, des doses de plus en plus fortes seront administrées jusqu'à l'obtention d'un effet optimum. Pour d'autres malades il peut être nécessaire d'administrer des doses d'entretien 1 à 8 fois par jour, de préférence 1 à 4 fois, selon les besoins physiologiques du malade considéré. Il est aussi possible que pour certains malades il soit nécessaire de n'utiliser qu'une à deux administrations journalières.

Chez l'homme, les doses sont généralement comprises entre 0,01 et 200 mg/kg. Par voie intrapéritonéale, les doses seront en général comprises entre 0,1 et 100 mg/kg et, de préférence entre 0,5 et 50 mg/kg et, encore plus spécifiquement entre 1 et 10 mg/kg. Par voie intraveineuse, les doses sont généralement comprises entre 0,1 et 50 mg/kg et, de préférence entre 0,1 et 5 mg/kg et, encore plus spécifquement entre 1 et 2 mg/kg. Il est entendu que, pour choisir le dosage le plus approprié, devront être pris en compte la voie d'administration, le poids du malade, son état de santé général, son âge et tous les facteurs qui peuvent influer sur l'efficacité du traitement.

L'exemple suivant illustre une composition selon l'invention.

### EXEMPLE

On dissout 40 mg du produit obtenu à l'exemple 1 dans 1 cm3 d'Emulphor EL 620 et 1 cm3 d'éthanol puis la solution est diluée par addition de 18 cm3 de sérum physiologique.

La composition est administrée par perfusion pendant 1 heure par introduction dans du soluté physiologique.

## Revendications

1. Nouveaux taxoïdes de formule générale : 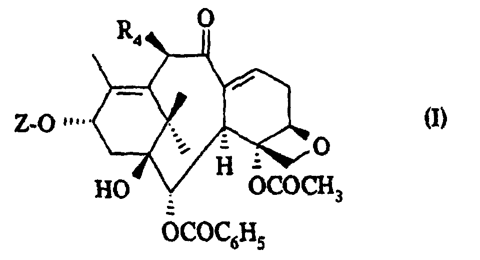 dans laquelle
Z représente un atome d'hydrogène ou un radical de formule générale: 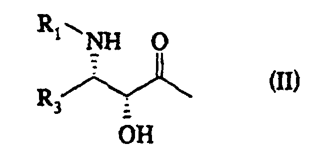 dans laquelle :
R₁ représente un radical benzoyle éventuellement substitué par un ou plusieurs atomes ou radicaux, identiques ou différents, choisis parmi les atomes d'halogène et les radicaux alcoyles contenant 1 à 4 atomes de carbone, alcoxy contenant 1 à 4 atomes de carbone ou trifluorométhyle, thénoyle ou furoyle ou un radical R₂-O-CO- dans lequel R₂ représente :
- un radical alcoyle contenant 1 à 8 atomes de carbone, alcényle contenant 2 à 8 atomes de carbone, alcynyle contenant 3 à 8 atomes de carbone, cycloalcoyle contenant 3 à 6 atomes de carbone, cycloalcényle contenant 4 à 6 atomes de carbone, bicycloalcoyle contenant 7 à 10 atomes de carbone, ces radicaux étant éventuellement substitués par un ou plusieurs substituants choisis parmi les atomes d'halogène et les radicaux hydroxy, alcoxy contenant 1 à 4 atomes de carbone, dialcoylamino dont chaque partie alcoyle contient 1 à 4 atomes de carbone, pipéridino, morpholino, pipérazinyl-1 (éventuellement substitué en -4 par un radical alcoyle contenant 1 à 4 atomes de carbone ou par un radical phénylalcoyle dont la partie alcoyle contient 1 à 4 atomes de carbone), cycloalcoyle contenant 3 à 6 atomes de carbone, cycloalcényle contenant 4 à 6 atomes de carbone, phényle (éventuellement substitué par un ou plusieurs atomes ou radicaux choisis parmi les atomes d'halogène et les radicaux alcoyles contenant 1 à 4 atomes de carbone ou alcoxy contenant 1 à 4 atomes de carbone), cyano, carboxy ou alcoxycarbonyle dont la partie alcoyle contient 1 à 4 atomes de carbone,
- un radical phényle ou α- ou β-naphtyle éventuellement substitué par un ou plusieurs atomes ou radicaux choisis parmi les atomes d'halogène et les radicaux alcoyles contenant 1 à 4 atomes de carbone ou alcoxy contenant 1 à 4 atomes de carbone ou un radical hétérocyclique aromatique à 5 chaînons choisi de préférence parmi les radicaux furyle et thiényle,
- ou un radical hétérocyclyle saturé contenant 4 à 6 atomes de carbone éventuellement substitué par un ou plusieurs radicaux alcoyles contenant 1 à 4 atomes de carbone,
R₃ représente un radical alcoyle droit ou ramifié contenant 1 à 8 atomes de carbone, alcényle droit ou ramifié contenant 2 à 8 atomes de carbone, alcynyle droit ou ramifié contenant 2 à 8 atomes de carbone, cycloalcoyle contenant 3 à 6 atomes de carbone, phényle ou α- ou β-naphtyle éventuellement substitué par un ou plusieurs atomes ou radicaux choisis parmi les atomes d'halogène et les radicaux alcoyles, alcényles, alcynyles, aryles, aralcoyles, alcoxy, alcoylthio, aryloxy, arylthio, hydroxy, hydroxyalcoyle, mercapto, formyle, acyle, acylamino, aroylamino, alcoxycarbonylamino, amino, alcoylamino, dialcoylamino, carboxy, alcoxycarbonyle, carbamoyle, alcoylcarbamoyle, dialcoylcarbamoyle, cyano, nitro et trifluorométhyle, ou un hétérocycle aromatique ayant 5 chaînons et contenant un ou plusieurs hétéroatomes, identiques ou différents, choisis parmi les atomes d'azote, d'oxygène ou de soufre et éventuellement substitué par un ou plusieurs substituants, identiques ou différents, choisis parmi les atomes d'halogène et les radicaux alcoyles, aryles, amino, alcoylamino, dialcoylamino, alcoxycarbonylamino, acyle, arylcarbonyle, cyano, carboxy, carbamoyle, alcoylcarbamoyle, dialcoylcarbamoyle ou alcoxycarbonyle, étant entendu que, dans les substituants des radicaux phényle, α- ou β-naphtyle et hétérocyclyles aromatiques, les radicaux alcoyles et les portions alcoyles des autres radicaux contiennent 1 à 4 atomes de carbone et que les radicaux alcényles et alcynyles contiennent 2 à 8 atomes de carbone et que les radicaux aryles sont des radicaux phényles ou α- ou β-naphtyles,
R₄ représente un atome d'hydrogène ou un radical hydroxy ou un radical alcoxy contenant 1 à 6 atomes de carbone en chaîne droite ou ramifiée, alcényloxy contenant 3 à 6 atomes de carbone en chaîne droite ou ramifiée, alcynyloxy contenant 3 à 6 atomes de carbone en chaîne droite ou ramifiée, cycloalcoyloxy contenant 3 à 6 atomes de carbone, cycloalcényloxy contenant 3 à 6 atomes de carbone, alcanoyloxy dont la partie alcanoyle contient 1 à 6 atomes de carbone en chaîne droite ou ramifiée, alcénoyloxy dont la partie alcénoyle contient 3 à 6 atomes de carbone en chaîne droite ou ramifiée, alcynoyloxy dont la partie alcynoyle contient 3 à 6 atomes de carbone en chaîne droite ou ramifiée, cycloalcanoyloxy contenant 1 à 6 atomes de carbone, alcoxyacétyle dont la partie alcoyle contient 1 à 6 atomes de carbone en chaîne droite ou ramifiée, alcoylthioacétyle dont la partie alcoyle contient 1 à 6 atomes de carbone en chaîne droite ou ramifiée, alcoyloxycarbonyloxy dont la partie alcoyle contient 1 à 6 atomes de carbone en chaîne droite ou ramifiée, ces radicaux étant éventuellement substitués par un ou plusieurs atomes d'halogène ou par un radical alcoxy contenant 1 à 4 atomes de carbone, alcoylthio contenant 1 à 4 atomes de carbone, ou un radical carboxy, alcoyloxycarbonyle dont la partie alcoyle contient 1 à 4 atomes de carbone, cyano, carbamoyle, N-alcoylcarbamoyle ou N,N-dialcoylcarbamoyle dont chaque partie alcoyle contient 1 à 4 atomes de carbone ou forme avec l'atome d'azote auquel elle est liée un radical hétérocyclique saturé contenant 5 ou 6 chaînons et éventuellement un second hétéroatome choisi parmi les atomes d'oxygène, de soufre ou d'azote éventuellement substitué par un radical alcoyle contenant 1 à 4 atomes de carbone ou un radical phényle ou un radical phénylalcoyle dont la partie alcoyle contient 1 à 4 atomes de carbone, ou bien R₄ représente un radical carbamoyloxy, alcoylcarbamoyloxy dont la partie alcoyle contient 1 à 4 atomes de carbone ou dialcoylcarbamoyloxy dont chaque partie alcoyle contient 1 à 4 atomes de carbone ou un radical benzoyloxy ou hétérocyclylcarbonyloxy dans lequel la partie hétérocyclique représente un hétérocycle aromatique 5 ou 6 chaînons contenant un ou plusieurs hétéroatomes choisis parmi les atomes d'oxygène, de soufre ou d'azote.

2. Nouveaux taxoïdes selon la revendication 1 pour lesquels Z représente un atome d'hydrogène ou un radical de formule générale (II) dans laquelle R₁ représente un radical benzoyle ou un radical R₂-O-CO- dans lequel R₂ représente un radical tert-butyle et R₃ représente un radical alcoyle contenant 1 à 6 atomes de carbone, alcényle contenant 2 à 6 atomes de carbone, cycloalcoyle contenant 3 à 6 atomes de carbone, phényle éventuellement substitué par un ou plusieurs atomes ou radicaux, identiques ou différents choisis parmi les atomes d'halogène (fluor, chlore) et les radicaux alcoyles (méthyle), alcoxy (méthoxy), dialcoylamino (diméthylamino), acylamino (acétylamino), alcoxycarbonylamino (tert-butoxycarbonylamino) ou trifluorométhyle ou un radical furyle-2 ou -3, thiényle-2 ou -3 ou thiazolyle-2, -4 ou -5 et R₄ représente un radical hydroxy ou un radical alcoyloxy droit ou ramifié contenant 1 à 6 atomes de carbone ou alcanoyloxy contenant 1 à 6 atomes de carbone.

3. Nouveaux taxoïdes selon la revendication 1 pour lesquels Z représente un atome d'hydrogène ou un radical de formule générale (II) dans laquelle R₁ représente un radical benzoyle ou un radical R₂-O-CO- dans lequel R₂ représente un radical tert-butyle et R₃ représente un radical isobutyle, isobutényle, butényle, cyclohexyle, phényle, furyle-2, furyle-3, thiényle-2, thiényle-3, thiazolyle-2, thiazolyle-4 ou thiazolyle-5, R₄ représente un radical hydroxy, méthoxy ou acétoxy.

4. Procédé de préparation d'un produit selon l'une des revendications 1, 2 ou 3 caractérisé en ce que l'on chauffe en présence d'un agent d'activation tel qu'un halogénure de métal alcalin ou un azoture de métal alcalin ou un sel d'ammonium ou de silice un produit de formule générale : 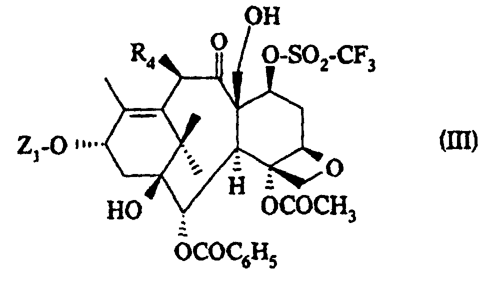 dans laquelle R₄ est défini comme précédemment et Z₁ représente un atome d'hydrogène ou un radical de formule générale : 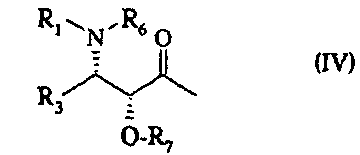 dans laquelle, ou bien, R₆ représente un atome d'hydrogène et R₇ représente un atome d'hydrogène ou un groupement protecteur de la fonction hydroxy, ou bien, R₆ et R₇ forment ensemble un hétérocycle saturé à 5 ou 6 chaînons, pour obtenir un produit de formule générale : 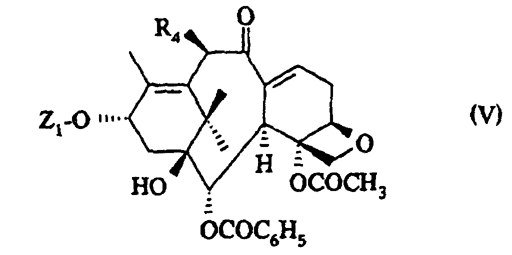 dans laquelle Z₁ et R₄ sont définis comme précédemment, suivi éventuellement du remplacement des groupements protecteurs par des atomes d'hydrogène.

5. Procédé selon la revendication 4 caractérisé en ce que l'on effectue la réaction d'élimination dans un solvant organique choisi parmi les éthers et les nitriles pris seuls ou en mélange à une température comprise entre 20°C et la température de reflux du mélange réactionnel.

6. Procédé selon la revendication 5 caractérisé en ce que l'on remplace les groupements protecteurs R₇ et/ou R₆ et R₇ par des atomes d'hydrogène en opérant, selon leur nature, de la manière suivante :
1) lorsque R₆ représente un atome d'hydrogène et R₇ représente un groupement protecteur de la fonction hydroxy, on remplace les groupements protecteurs par des atomes d'hydrogène au moyen d'un acide minéral ou organique utilisé seul ou en mélange en opérant dans un solvant organique choisi parmi les alcools, les éthers, les esters, les hydrocarbures aliphatiques, les hydrocarbures aliphatiques halogénés, les hydrocarbures aromatiques ou les nitriles à une température comprise entre -10 et 60°C, ou au moyen d'une source d'ions fluorures ou par hydrogénation catalytique
2) lorsque R₆ et R₇ forment ensemble un hétérocycle saturé à 5 ou 6 chaînons et plus particulièrement un cycle oxazolidine de formule générale : 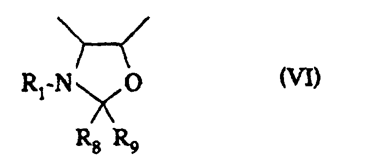 dans laquelle R₁ est défini comme précédemment, R₈ et R₉, identiques ou différents, représentent un atome d'hydrogène ou un radical alcoyle contenant 1 à 4 atomes de carbone, ou un radical aralcoyle dont la partie alcoyle contient 1 à 4 atomes de carbone et la partie aryle représente un radical phényle éventuellement substitué par un ou plusieurs radicaux alcoxy contenant 1 à 4 atomes de carbone, ou un radical aryle représentant un radical phényle éventuellement substitué par un ou plusieurs radicaux alcoxy contenant 1 à 4 atomes de carbone, ou bien R₈ représente un radical alcoxy contenant 1 à 4 atomes de carbone ou un radical trihalométhyle tel que trichlorométhyle ou un radical phényle substitué par un radical trihalométhyle tel que trichlorométhyle et R₉ représente un atome d'hydrogène, ou bien R₈ et R₉ forment ensemble avec l'atome de carbone auquel ils sont liés un cycle ayant 4 à 7 chaînons, on remplace le groupement protecteur formé par R₆ et R₇ par des atomes d'hydrogène en opérant, selon les significations de R₁, R₈ et R₉, de la manière suivante :
a) lorsque R₁ représente un radical tert-butoxycarbonyle, R₈ et R₉, identiques ou différents, représentent un radical alcoyle ou un radical aralcoyle ou aryle, ou bien R₈ représente un radical trihalométhyle ou un radical phényle substitué par un radical trihalométhyle, et R₉ représente un atome d'hydrogène, ou bien R₈ et R₉ forment ensemble un cycle ayant de 4 à 7 chaînons, on traite l'ester de formule générale (V) par un acide minéral ou organique éventuellement dans un solvant organique tel qu'un alcool pour obtenir un produit de formule générale : 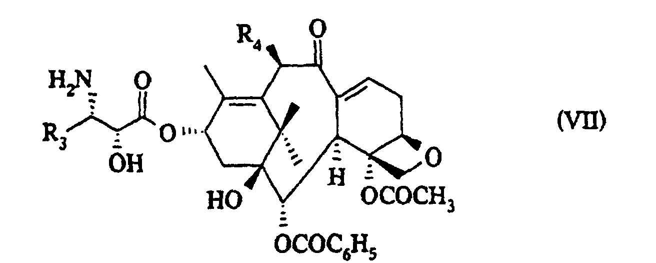 dans laquelle R₃ et R₄ sont définis comme précédemment, que l'on acyle au moyen de chlorure de benzoyle dans lequel le noyau phényle est éventuellemnt substitué, de chlorure de thénoyle, de chlorure de furoyle ou d'un produit de formule générale :
R₂-O-CO-X (VIII)
dans laquelle R₂ est défini comme précédemment et X représente un atome d'halogène ou un reste -O-R₂ ou -O-CO-O-R₂, pour obtenir un produit de formule générale (I) dans laquelle Z représente un radical de formule générale (II).
b) lorsque R₁ représente un radical benzoyle éventuellement substitué, thénoyle ou furoyle ou un radical R₂O-CO- dans lequel R₂ est défini comme précédemment, R₈ représente un atome d'hydrogène ou un radical alcoxy contenant 1 à 4 atomes de carbone ou un radical phényle substitué par un ou plusieurs radicaux alcoxy contenant 1 à 4 atomes de carbone et R₉ représente un atome d'hydrogène, on remplace le groupement protecteur formé par R₆ et R₇ par des atomes d'hydrogène en opérant en présence d'un acide minéral ou organique utilisé seul ou en mélange en quantité stoechiométrique ou catalytique, en opérant dans un solvant organique choisi parmi les alcools, les éthers, les esters, les hydrocarbures aliphatiques, les hydrocarbures aliphatiques halogénés et les hydrocarbures aromatiques à une température comprise entre -10 et 60°C.

7. Composition pharmaceutique caractérisée en ce qu'elle contient au moins un produit selon l'une des revendications 1, 2 ou 3 pour lequel Z représente un radical de formule générale (II) en association avec un ou plusieurs diluants ou adjuvants pharmaceutiquement acceptables et éventuellement un ou plusieurs composés compatibles et pharmacologiquement actifs.

## Claims

1. New taxoids of general formula: 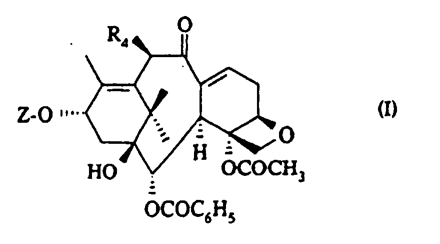 in which:
Z represents a hydrogen atom or a radical of general formula: 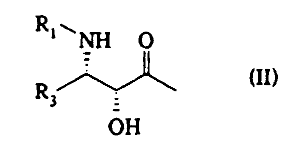 in which:
R₁ represents a benzoyl radical optionally substituted with one or more identical or different atoms or radicals chosen from halogen atoms and alkyl radicals containing 1 to 4 carbon atoms, alkoxy radicals containing 1 to 4 carbon atoms or trifluoromethyl radicals, a thenoyl or furoyl radical or a radical R₂-O-CO- in which R₂ represents:
- an alkyl radical containing 1 to 8 carbon atoms, an alkenyl radical containing 2 to 8 carbon atoms, an alkynyl radical containing 3 to 8 carbon atoms, a cycloalkyl radical containing 3 to 6 carbon atoms, a cycloalkenyl radical containing 4 to 6 carbon atoms or a bicycloalkyl radical containing 7 to 10 carbon atoms, these radicals being optionally substituted with one or more substituents chosen from halogen atoms and hydroxyl radicals, alkoxy radicals containing 1 to 4 carbon atoms, dialkylamino radicals in which each alkyl portion contains 1 to 4 carbon atoms, piperidino or morpholino radicals, 1-piperazinyl radicals (optionally substituted at position 4 with an alkyl radical containing 1 to 4 carbon atoms or with a phenylalkyl radical in which the alkyl portion contains 1 to 4 carbon atoms), cycloalkyl radicals containing 3 to 6 carbon atoms, cycloalkenyl radicals containing 4 to 6 carbon atoms, phenyl radicals (optionally substituted with one or more atoms or radicals chosen from halogen atoms and alkyl radicals containing 1 to 4 carbon atoms or alkoxy radicals containing 1 to 4 carbon atoms), cyano or carboxyl radicals or alkoxycarbonyl radicals in which the alkyl portion contains 1 to 4 carbon atoms,
- a phenyl or α- or β-naphthyl radical optionally substituted with one or more atoms or radicals chosen from halogen atoms and alkyl radicals containing 1 to 4 carbon atoms or alkoxy radicals containing 1 to 4 carbon atoms, or a 5-membered aromatic heterocyclic radical preferably chosen from furyl and thienyl radicals,
- or a saturated heterocyclic radical containing 4 to 6 carbon atoms, optionally substituted with one or more alkyl radicals containing 1 to 4 carbon atoms,
R₃ represents an unbranched or branched alkyl radical containing 1 to 8 carbon atoms, an unbranched or branched alkenyl radical containing 2 to 8 carbon atoms, an unbranched or branched alkynyl radical containing 2 to 8 carbon atoms, a cycloalkyl radical containing 3 to 6 carbon atoms, a phenyl or α- or β-naphthyl radical optionally substituted with one or more atoms or radicals chosen from halogen atoms and alkyl, alkenyl, alkynyl, aryl, aralkyl, alkoxy, alkylthio, aryloxy, arylthio, hydroxyl, hydroxyalkyl, mercapto, formyl, acyl, acylamino, aroylamino, alkoxycarbonylamino, amino, alkylamino, dialkylamino, carboxyl, alkoxycarbonyl, carbamoyl, alkylcarbamoyl, dialkylcarbamoyl, cyano, nitro and trifluoromethyl radicals, or a 5-membered aromatic heterocycle containing one or more identical or different hetero atoms chosen from nitrogen, oxygen and sulphur atoms and optionally substituted with one or more identical or different substituents chosen from halogen atoms and alkyl, aryl, amino, alkylamino, dialkylamino, alkoxycarbonylamino, acyl, arylcarbonyl, cyano, carboxyl, carbamoyl, alkylcarbamoyl, dialkylcarbamoyl or alkoxycarbonyl radicals, on the understanding that, in the substituents of the phenyl, α- or β-naphthyl and aromatic heterocyclic radicals, the alkyl radicals and the alkyl portions of the other radicals contain 1 to 4 carbon atoms, and that the alkenyl and alkynyl radicals contain 2 to 8 carbon atoms, and that the aryl radicals are phenyl or α- or β-naphthyl radicals,
R₄ represents a hydrogen atom or a hydroxyl radical or an alkoxy radical containing 1 to 6 carbon atoms in an unbranched or branched chain, an alkenyloxy radical containing 3 to 6 carbon atoms in an unbranched or branched chain, an alkynyloxy radical containing 3 to 6 carbon atoms in an unbranched or branched chain, a cycloalkyloxy radical containing 3 to 6 carbon atoms, a cycloalkenyloxy radical containing 3 to 6 carbon atoms, an alkanoyloxy radical in which the alkanoyl portion contains 1 to 6 carbon atoms in an unbranched or branched chain, an alkenoyloxy radical in which the alkenoyl portion contains 3 to 6 carbon atoms in an unbranched or branched chain, an alkynoyloxy radical in which the alkynoyl portion contains 3 to 6 carbon atoms in an unbranched or branched chain, a cycloalkanoyloxy radical containing 1 to 6 carbon atoms, an alkoxyacetyl radical in which the alkyl portion contains 1 to 6 carbon atoms in an unbranched or branched chain, an alkylthioacetyl radical in which the alkyl portion contains 1 to 6 carbon atoms in an unbranched or branched chain or an alkyloxycarbonyloxy radical in which the alkyl portion contains 1 to 6 carbon atoms in an unbranched or branched chain, these radicals being optionally substituted with one or more halogen atoms or with an alkoxy radical containing 1 to 4 carbon atoms, an alkylthio radical containing 1 to 4 carbon atoms or a carboxyl radical, an alkyloxycarbonyl radical in which the alkyl portion contains 1 to 4 carbon atoms, a cyano or carbamoyl radical or an N-alkylcarbamoyl or N,N-dialkylcarbamoyl radical in which each alkyl portion contains 1 to 4 carbon atoms or, with the nitrogen atom to which it is linked, forms a saturated 5- or 6-membered heterocyclic radical optionally containing a second hetero atom chosen from oxygen, sulphur and nitrogen atoms, optionally substituted with an alkyl radical containing 1 to 4 carbon atoms or a phenyl radical or a phenylalkyl radical in which the alkyl portion contains 1 to 4 carbon atoms, or alternatively R₄ represents a carbamoyloxy radical, an alkylcarbamoyloxy radical in which the alkyl portion contains 1 to 4 carbon atoms, a dialkylcarbamoyloxy radical in which each alkyl portion contains 1 to 4 carbon atoms, a benzoyloxy radical or a heterocyclic radical attached to a carbonyloxy group in which radical the heterocyclic portion represents a 5- or 6-membered aromatic heterocycle containing one or more hetero atoms chosen from oxygen, sulphur and nitrogen atoms.

2. New taxoids according to claim 1 for which Z represents a hydrogen atom or a radical of general formula (II) in which R₁ represents a benzoyl radical or a radical R₂-O-CO- in which R₂ represents a tert-butyl radical and R₃ represents an alkyl radical containing 1 to 6 carbon atoms, an alkenyl radical containing 2 to 6 carbon atoms, a cycloalkyl radical containing 3 to 6 carbon atoms, a phenyl radical optionally substituted with one or more identical or different atoms or radicals chosen from halogen atoms (fluorine, chlorine) and alkyl (methyl), alkoxy (methoxy), dialkylamino (dimethylamino), acylamino (acetylamino), alkoxycarbonylamino (tert-butoxycarbonylamino) or trifluoromethyl radicals, or a 2- or 3-furyl, 2- or 3-thienyl or 2-, 4- or 5-thiazolyl radical, and R₄ represents a hydroxyl radical or an unbranched or branched alkyloxy radical containing 1 to 6 carbon atoms or an alkanoyloxy radical containing 1 to 6 carbon atoms.

3. New taxoids according to claim 1 for which Z represents a hydrogen atom or a radical of general formula (II) in which R₁ represents a benzoyl radical or a radical R₂-O-CO- in which R₂ represents a tert-butyl radical and R₃ represents an isobutyl, isobutenyl, butenyl, cyclohexyl, phenyl, 2-furyl, 3-furyl, 2-thienyl, 3-thienyl, 2-thiazolyl, 4-thiazolyl or 5-thiazolyl radical, and R₄ represents a hydroxyl, methoxy or acetoxy radical.

4. Process for preparing a product according to one of claims 1, 2 and 3, characterized in that a product of general formula: 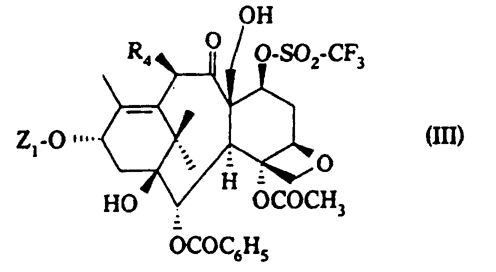 in which R₄ is defined as above and Z₁ represents a hydrogen atom or a radical of general formula: 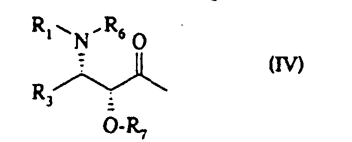 in which either R₆ represents a hydrogen atom and R₇ represents a hydrogen atom or a group protecting the hydroxyl function, or R₆ and R₇ together form a saturated 5- or 6-membered heterocycle, is heated in the presence of an activating agent such as an alkali metal halide or an alkali metal azide or an ammonium salt or silica to obtain a product of general formula: 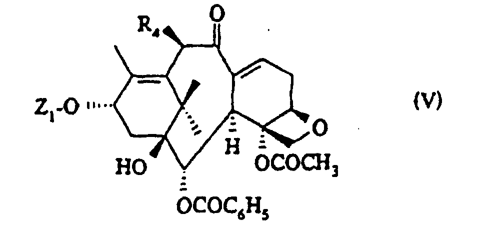 in which Z₁ and R₄ are defined as above, followed where appropriate by replacement of the protective groups by hydrogen atoms.

5. Process according to claim 4, characterized in that the elimination reaction is performed in an organic solvent chosen from ethers and nitriles, taken alone or mixed, at a temperature between 20°C and the refluxing temperature of the reaction mixture.

6. Process according to claim 5, characterized in that the protective groups R₇ and/or R₆ and R₇ are replaced by hydrogen atoms, working, depending on their nature, in the following manner:
1) when R₆ represents a hydrogen atom and R₇ represents a group protecting the hydroxyl function, the protective groups are replaced by hydrogen atoms by means of an inorganic or organic acid used alone or mixed, working in an organic solvent chosen from alcohols, ethers, esters, aliphatic hydrocarbons, halogenated aliphatic hydrocarbons, aromatic hydrocarbons or nitriles at a temperature of between -10 and 60°C, or by means of a source of fluoride ions, or by catalytic hydrogenation,
2) when R₆ and R₇ together form a saturated 5- or 6-membered heterocycle, and more especially an oxazolidine ring of general formula: 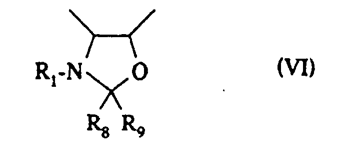 in which R₁ is defined as above and R₈ and R₉, which may be identical or different, represent a hydrogen atom or an alkyl radical containing 1 to 4 carbon atoms, or an aralkyl radical in which the alkyl portion contains 1 to 4 carbon atoms and the aryl portion represents a phenyl radical optionally substituted with one or more alkoxy radicals containing 1 to 4 carbon atoms, or an aryl radical representing a phenyl radical optionally substituted with one or more alkoxy radicals containing 1 to 4 carbon atoms, or alternatively R₈ represents an alkoxy radical containing 1 to 4 carbon atoms or a trihalomethyl radical such as trichloromethyl or a phenyl radical substituted with a trihalomethyl radical such as trichloromethyl and R₉ represents a hydrogen atom, or alternatively R₈ and R₉, together with the carbon atom to which they are linked, form a 4- to 7-membered ring, the protective group formed by R₆ and R₇ is replaced by hydrogen atoms, working, depending on the meanings of R₁, R₈ and R₉, in the following manner:
a) when R₁ represents a tert-butoxycarbonyl radical and R₈ and R₉, which may be identical or different, represent an alkyl radical or an aralkyl or aryl radical, or alternatively R₈ represents a trihalomethyl radical or a phenyl radical substituted with a trihalomethyl radical and R₉ represents a hydrogen atom, or alternatively R₈ and R₉ together form a 4- to 7-membered ring, the ester of general formula (V) is treated with an inorganic or organic acid, where appropriate in an organic solvent such as an alcohol, to obtain a product of general formula: 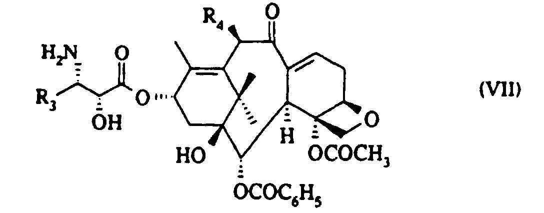 in which R₃ and R₄ are defined as above, which is acylated by means of benzoyl chloride in which the phenyl ring is optionally substituted or by means of thenoyl chloride, of furoyl chloride or of a product of general formula:
R₂-O-CO-X (VIII)
in which R₂ is defined as above and X represents a halogen atom or a residue -O-R₂ or -O-CO-O-R₂, to obtain a product of general formula (I) in which Z represents a radical of general formula (II),
b) when R₁ represents an optionally substituted benzoyl radical, a thenoyl or furoyl radical or a radical R₂O-CO- in which R₂ is defined as above, R₈ represents a hydrogen atom or an alkoxy radical containing 1 to 4 carbon atoms or a phenyl radical substituted with one or more alkoxy radicals containing 1 to 4 carbon atoms and R₉ represents a hydrogen atom, the protective group formed by R₆ and R₇ is replaced by hydrogen atoms, working in the presence of an inorganic or organic acid used alone or mixed in a stoichiometric or catalytic amount, working in an organic solvent chosen from alcohols, ethers, esters, aliphatic hydrocarbons, halogenated aliphatic hydrocarbons and aromatic hydrocarbons at a temperature of between -10 and 60°C.

7. Pharmaceutical composition, characterized in that it contains at least one product according to one of claims 1, 2 and 3 for which Z represents a radical of general formula (II), in combination with one or more pharmaceutically acceptable diluents or adjuvants and optionally one or more compatible and pharmacologically active compounds.

## Patentansprüche

1. Neue Taxoide der allgemeinen Formel (I) 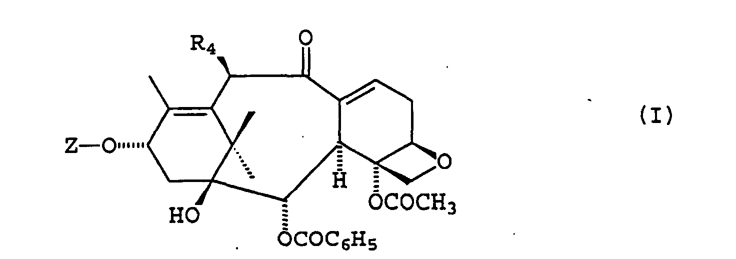 in der
Z ein Wasserstoffatom oder einen Rest der allgemeinen Formel (II) 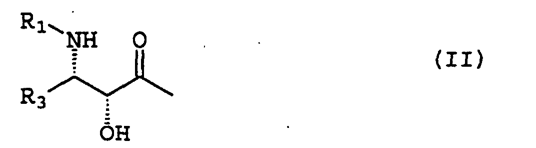 darstellt, worin
R₁ einen Rest Benzoyl, gegebenenfalls substituiert durch ein oder mehrere, gleiche oder verschiedene Atome oder Reste, ausgewählt unter den Halogenatomen und den Resten Alkyl mit 1 bis 4 Kohlenstoffatomen, Alkoxy mit 1 bis 4 Kohlenstoffatomen oder Trifluormethyl, Thenoyl oder Furoyl oder einem Rest R₂-O-CO-, bedeutet, worin R₂ darstellt:
- einen Rest Alkyl mit 1 bis 8 Kohlenstoffatomen, Alkenyl mit 2 bis 8 Kohlenstoffatomen, Alkinyl mit 3 bis 8 Kohlenstoffatomen, Cycloalkyl mit 3 bis 6 Kohlenstoffatomen, Cycloalkenyl mit 4 bis 6 Kohlenstoffatomen, Bicycloalkyl mit 7 bis 10 Kohlenstoffatomen, wobei diese Reste gegebenenfalls substituiert sind durch einen oder mehrere Substituenten, ausgewählt unter den Halogenatomen und den Resten Hydroxy, Alkoxy mit 1 bis 4 Kohlenstoffatomen, Dialkylamino, bei dem jeder Teil Alkyl 1 bis 4 Kohlenstoffatome enthält, Piperidino, Morpholino, 1-Piperazinyl (gegebenenfalls substituiert in -4 durch einen Rest Alkyl mit 1 bis 4 Kohlenstoffatomen oder durch einen Rest Phenylalkyl, dessen Teil Alkyl 1 bis 4 Kohlenstoffatome enthält), Cycloalkyl mit 3 bis 6 Kohlenstoffatomen, Cycloalkenyl mit 4 bis 6 Kohlenstoffatomen, Phenyl (gegebenenfalls substituiert durch ein oder mehrere Atome oder Reste, ausgewählt unter den Halogenatomen und den Resten Alkyl mit 1 bis 4 Kohlenstoffatomen oder Alkoxy mit 1 bis 4 Kohlenstoffatomen), Cyano, Carboxy oder Alkoxycarbonyl, dessen Teil Alkyl 1 bis 4 Kohlenstoffatome enthält,
- einen Rest Phenyl oder α- oder β-Naphthyl, gegebenenfalls substituiert durch ein oder mehrere Atome oder Reste, ausgewählt unter den Halogenatomen und den Resten Alkyl mit 1 bis 4 Kohlenstoffatomen oder Alkoxy mit 1 bis 4 Kohlenstoffatomen oder einem aromatischen heterocyclischen Rest mit 5 Ringgliedern, vorzugsweise ausgewählt unter den Resten Furyl und Thienyl,
- oder einem gesättigten heterocyclischen Rest mit 4 bis 6 Kohlenstoffatomen, gegebenenfalls substituiert durch einen oder mehrere Alkylreste mit 1 bis 4 Kohlenstoffatomen,
- R₃ einen geraden oder verzweigten Rest Alkyl mit 1 bis 8 Kohlenstoffatomen, einen geraden oder verzweigten Rest Alkenyl mit 2 bis 8 Kohlenstoffatomen, einen geraden oder verzweigten Rest Alkinyl mit 2 bis 8 Kohlenstoffatomen, Cycloalkyl mit 3 bis 6 Kohlenstoffatomen, Phenyl oder α- oder β-Naphthyl darstellt, gegebenenfalls substituiert durch ein oder mehrere Atome oder Reste, ausgewählt unter den Halogenatomen und den Resten Alkyl, Alkenyl, Alkinyl, Aryl, Aralkyl, Alkoxy, Alkylthio, Aryloxy, Arylthio, Hydroxy, Hydroxyalkyl, Mercapto, Formyl, Acyl, Acylamino, Aroylamino, Alkoxycarbonylamino, Amino, Alkylamino, Dialkylamino, Carboxy, Alkoxycarbonyl, Carbamoyl, Alkylcarbamoyl, Dialkylcarbamoyl, Cyano, Nitro und Trifluormethyl, oder einen aromatischen Heterocyclus mit 5 Ringgliedern und einem oder mehreren, gleichen oder verschiedenen Heteroatomen bedeutet, ausgewählt unter den Atomen von Stickstoff, Sauerstoff oder Schwefel, und gegebenenfalls substituiert durch einen oder mehrere gleiche oder verschiedene Substituenten, ausgewählt unter den Halogenatomen und den Resten Alkyl, Aryl, Amino, Alkylamino, Dialkylamino, Alkoxycarbonylamino, Acyl, Arylcarbonyl, Cyano, Carboxy, Carbamoyl, Alkylcarbamoyl, Dialkylcarbamoyl oder Alkoxycarbonyl, mit der Maßgabe, daß in den Substituenten der Reste Phenyl, α- oder β-Naphthyl und von aromatischem Heterocyclus die Reste Alkyl und die Teile Alkyl der anderen Reste 1 bis 4 Kohlenstoffatome enthalten und daß die Reste Alkenyl und Alkinyl 2 bis 8 Kohlenstoffatome enthalten und daß die Reste Aryl Reste Phenyl oder α- oder β-Naphthyl sind,
R₄ ein Wasserstoffatom oder einen Rest Hydroxy oder einen Rest Alkoxy mit 1 bis 6 Kohlenstoffatomen in gerader oder verzweigter Kette, Alkenyloxy mit 3 bis 6 Kohlenstoffatomen in gerader oder verzweigter Kette, Alkinyloxy mit 3 bis 6 Kohlenstoffatomen in gerader oder verzweigter Kette, Cycloalkyloxy mit 3 bis 6 Kohlenstoffatomen, Cycloalkenyloxy mit 3 bis 6 Kohlenstoffatomen, Alkanoyloxy, dessen Teil Alkanoyl 1 bis 6 Kohlenstoffatome in gerader oder verzweigter Kette enthält, Alkenoyloxy, dessen Teil Alkenoyl 3 bis 6 Kohlenstoffatome in gerader oder verzweigter Kette enthält, Alkinoyloxy, dessen Teil Alkinoyl 3 bis 6 Kohlenstoffatome in gerader oder verzweigter Kette enthält, Cycloalkanoyloxy mit 1 bis 6 Kohlenstoffatomen, Alkoxyacetyl, dessen Teil Alkyl 1 bis 6 Kohlenstoffatome in gerader oder verzweigter Kette enthält, Alkylthioacetyl, dessen Teil Alkyl 1 bis 6 Kohlenstoffatome in gerader oder verzweigter Kette enthält, Alkyloxycarbonyloxy, dessen Teil Alkyl 1 bis 6 Kohlenstoffatome in gerader oder verzweigter Kette enthält, darstellt, wobei diese Reste gegebenenfalls substituiert sind durch ein oder mehrere Halogenatome oder durch einen Rest Alkoxy mit 1 bis 4 Kohlenstoffatomen, Alkylthio mit 1 bis 4 Kohlenstoffatomen, oder einen Rest Carboxy, Alkyloxycarbonyl, dessen Teil Alkyl 1 bis 4 Kohlenstoffatome enthält, Cyano, Carbamoyl, N-Alkylcarbamoyl oder N,N-Dialkylcarbamoyl, bei dem jeder Teil Alkyl 1 bis 4 Kohlenstoffatome enthält oder zusammen mit dem Stickstoffatom, an das es gebunden ist, einen gesättigten heterocyclischen Rest mit 5 oder 6 Ringgliedern und gegebenenfalls einem zweiten Heteroatom bildet, ausgewählt unter den Atomen von Sauerstoff, Schwefel oder Stickstoff, gegebenenfalls substituiert durch einen Rest Alkyl mit 1 bis 4 Kohlenstoffatomen oder einen Rest Phenyl oder einen Rest Phenylalkyl, dessen Teil Alkyl 1 bis 4 Kohlenstoffatome enthält, oder R₄ auch einen Rest Carbamoyloxy, Alkylcarbamoyloxy, dessen Teil Alkyl 1 bis 4 Kohlenstoffatome enthält, oder Dialkylcarbamoyloxy, bei dem jeder Teil Alkyl 1 bis 4 Kohlenstoffatome enthält, oder einen Rest Benzoyloxy oder Heterocyclylcarbonyloxy darstellt, worin der heterocyclische Teil einen aromatischen Heterocyclus mit 5 oder 6 Ringgliedern und einem oder mehreren Heteroatomen, ausgewählt unter den Atomen von Sauerstoff, Schwefel oder Stickstoff, bedeutet.

2. Neue Taxoide nach Anspruch 1, worin Z ein Wasserstoffatom oder einen Rest der allgemeinen Formel (II) darstellt, in der R₁ einen Rest Benzoyl oder einen Rest R₂-O-CO- bedeutet, worin R₂ ein Rest tert.-Butyl ist und R₃ einen Rest Alkyl mit 1 bis 6 Kohlenstoffatomen, Alkenyl mit 2 bis 6 Kohlenstoffatomen, Cycloalkyl mit 3 bis 6 Kohlenstoffatomen, Phenyl, gegebenenfalls substituiert durch ein oder mehrere, gleiche oder verschiedene Atome oder Reste, ausgewählt unter den Halogenatomen (Fluor, Chlor) und den Resten Alkyl (Methyl), Alkoxy (Methoxy), Dialkylamino (Dimethylamino), Acylamino (Acetylamino), Alkoxycarbonylamino (tert.-Butoxycarbonylamino) oder Trifluormethyl, oder einen Rest 2-Furyl oder 3-Furyl, 2-Thienyl oder 3-Thienyl oder 2-Thiazolyl, 4-Thiazolyl oder 5-Thiazolyl darstellt und R₄ einen Rest Hydroxy oder einen geraden oder verzweigten Rest Alkyloxy mit 1 bis 6 Kohlenstoffatomen oder Alkanoyloxy mit 1 bis 6 Kohlenstoffatomen bedeutet.

3. Neue Taxoide nach Anspruch 1, worin Z ein Wasserstoffatom oder einen Rest der allgemeinen Formel (II) darstellt, in der R₁ einen Rest Benzoyl oder einen Rest R₂-O-CO- bedeutet, worin R₂ ein Rest tert.-Butyl ist und R₃ einen Rest Isobutyl, Isobutenyl, Butenyl, Cyclohexyl, Phenyl, 2-Furyl, 3-Furyl, 2-Thienyl, 3-Thienyl, 2-Thiazolyl, 4-Thiazolyl oder 5-Thiazolyl darstellt und R₄ einen Rest Hydroxy, Methoxy oder Acetoxy bedeutet.

4. Verfahren zur Herstellung eines Produktes nach einem der Ansprüche 1, 2 oder 3, dadurch gekennzeichnet, daß man in Anwesenheit eines Aktivierungsmittels wie einem Alkalimetallhalogenid oder einem Alkalimetallazid oder einem Ammoniumsalz oder Siliciumdioxid ein Produkt der allgemeinen Formel (III) 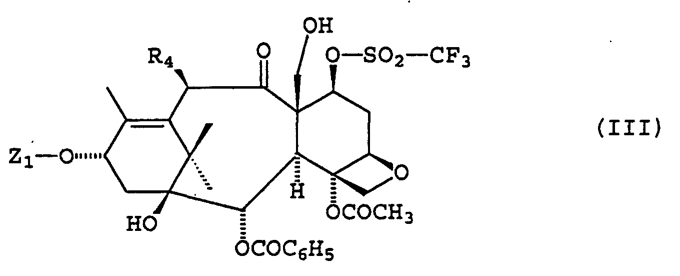 erhitzt, in der R₄ wie oben definiert ist und Z₁ ein Wasserstoffatom oder einen Rest der allgemeinen Formel (IV) 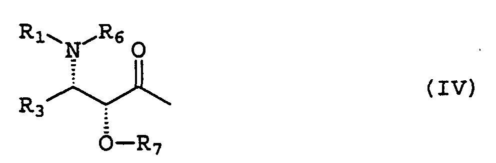 darstellt, in der entweder R₆ ein Wasserstoffatom ist und R₇ ein Wasserstoffatom oder eine Schutzgruppe für die Hydroxyfunktion darstellt, oder R₆ und R₇ zusammen einen gesättigten Heterocyclus mit 5 oder 6 Ringgliedern bilden, um ein Produkt der allgemeinen Formel (V) 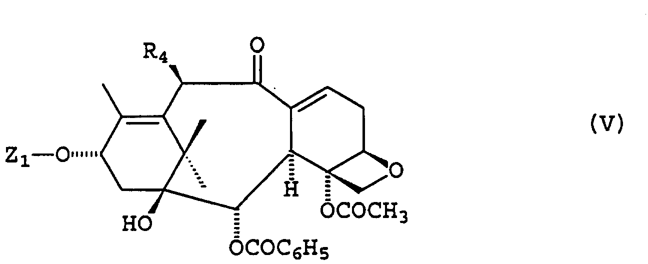 zu erhalten, in der Z₁ und R₄ wie oben definiert sind, gegebenenfalls gefolgt von einem Austausch der Schutzgruppen durch Wasserstoffatome.

5. Verfahren nach Anspruch 4, dadurch gekennzeichnet, daß man die Entfernungsreaktion in einem organischen Lösungsmittel, ausgewählt unter den Ethern und den Nitrilen, allein verwendet oder in Mischung, und bei einer Temperatur zwischen 20 °C und der Rückflußtemperatur der Reaktionsmischung durchführt.

6. Verfahren nach Anspruch 5, dadurch gekennzeichnet, daß man den Austausch der Schutzgruppen R₇ und/oder R₆ und R₇ durch Wasserstoffatome durchführt, indem man je nach ihrer Beschaffenheit wie folgt arbeitet:
1) wenn R₆ ein Wasserstoffatom ist und R₇ eine Schutzgruppe für die Hydroxyfunktion darstellt, nimmt man den Austausch der Schutzgruppen durch Wasserstoffatome mit Hilfe einer Mineralsäure oder organischen Säure vor, allein verwendet oder in Mischung, wobei man in einem organischen Lösungsmittel, ausgewählt unter den Alkoholen, den Ethern, den Estern, den aliphatischen Kohlenwasserstoffen, den halogenierten aliphatischen Kohlenwasserstoffen, den aromatischen Kohlenwasserstoffen oder den Nitrilen, und bei einer Temperatur zwischen -10 °C und 60 °C arbeitet, oder auch mit Hilfe einer Quelle von Fluoridionen oder durch katalytische Hydrierung,
2) wenn R₆ und R₇ zusammen einen gesättigten Heterocyclus mit 5 oder 6 Ringgliedern bilden und ganz besonders einen Oxazolidin-Ring der allgemeinen Formel (VI) 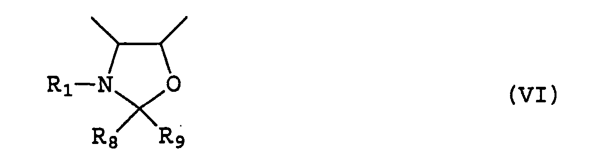 in der R₁ wie oben definiert ist, R₈ und R₉, gleich oder verschieden, ein Wasserstoffatom oder einen Rest Alkyl mit 1 bis 4 Kohlenstoffatomen oder einen Rest Aralkyl, dessen Teil Alkyl 1 bis 4 Kohlenstoffatome enthält und dessen Teil Aryl ein Rest Phenyl ist, gegebenenfalls substituiert durch einen oder mehrere Reste Alkoxy mit 1 bis 4 Kohlenstoffatomen, oder einen Rest Aryl darstellen, der ein Rest Phenyl ist, gegebenenfalls substituiert durch einen oder mehrere Reste Alkoxy mit 1 bis 4 Kohlenstoffatomen, oder auch R₈ einen Rest Alkoxy mit 1 bis 4 Kohlenstoffatomen oder einen Rest Trihalomethyl wie Trichlormethyl, oder einen Rest Phenyl bedeutet, substituiert durch einen Rest Trihalomethyl wie Trichlormethyl, und R₉ ein Wasserstoffatom ist, oder auch R₈ und R₉ zusammen mit dem Kohlenstoffatom, an das sie gebunden sind, einen Ring mit 4 bis 7 Ringgliedern bilden, nimmt man den Austausch der durch R₆ und R₇ gebildeten Schutzgruppe durch Wasserstoffatome vor, indem man je nach den Bedeutungen von R₁, R₈ und R₉ in der folgenden Weise arbeitet:
a) wenn R₁ ein Rest tert.-Butoxycarbonyl ist, R₈ und R₉, gleich oder verschieden, einen Rest Alkyl oder einen Rest Aralkyl oder Aryl darstellen, oder auch R₈ einen Rest Trihalomethyl oder einen Rest Phenyl, substituiert durch einen Rest Trihalomethyl, bedeutet und R₉ ein Wasserstoffatom ist, oder auch R₈ und R₉ zusammen einen Ring mit 4 bis 7 Ringgliedern bilden, so behandelt man den Ester der allgemeinen Formel (V) durch eine Mineralsäure oder organische Säure, gegebenenfalls in einem organischen Lösungsmittel wie einem Alkohol, um ein Produkt der allgemeinen Formel (VII) 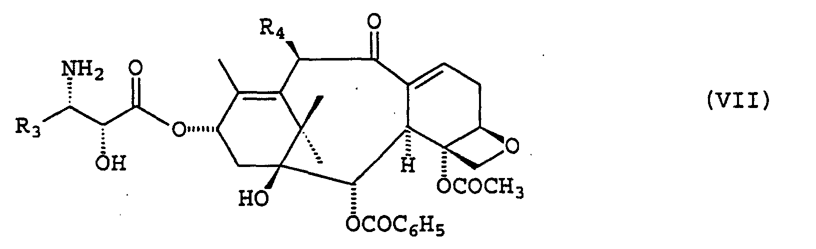 zu erhalten, in der R₃ und R₄ wie oben definiert sind, das man dann mit Hilfe von Benzoylchlorid, worin der Phenylkern gegebenenfalls substituiert ist, von Thenoylchlorid, von Furoylchlorid oder einem Produkt der allgemeinen Formel (VIII)
R₂-O-CO-X (VIII)
acyliert, in der R₂ wie oben definiert ist und X ein Halogenatom oder einen Rest -O-R₂ oder -O-CO-O-R₂ bedeutet, um ein Produkt der allgemeinen Formel (I) zu erhalten, worin Z einen Rest der allgemeinen Formel (II) darstellt;
b) wenn R₁ einen Rest Benzoyl, gegebenenfalls substituiert, Thenoyl oder Furoyl oder einen Rest R₂O-CO- darstellt, worin R₂ wie oben definiert ist, R₈ ein Wasserstoffatom oder einen Rest Alkoxy mit 1 bis 4 Kohlenstoffatomen oder einen Rest Phenyl bedeutet, substituiert durch einen oder mehrere Reste Alkoxy mit 1 bis 4 Kohlenstoffatomen, und R₉ ein Wasserstoffatom ist, nimmt man den Austausch der durch R₆ und R₇ gebildeten Schutzgruppen durch Wasserstoffatome vor, indem man in Anwesenheit einer Mineralsäure oder organischen Säure, allein verwendet oder in Mischung stöchiometrischer oder katalytischer Mengen, sowie in einem organischen Lösungsmittel, ausgewählt unter den Alkoholen, den Ethern, den Estern, den aliphatischen Kohlenwasserstoffen, den halogenierten aliphatischen Kohlenwasserstoffen und den aromatischen Kohlenwasserstoffen und bei einer Temperatur zwischen -10 °C und 60 °C arbeitet.

7. Pharmazeutische Zusammensetzung, dadurch gekennzeichnet, daß sie mindestens ein Produkt nach einem der Ansprüche 1, 2 oder 3, worin Z ein Rest der allgemeinen Formel (II) ist, in Assoziation mit einem oder mehreren pharmazeutisch akzeptablen Verdünnungsmitteln oder Zusatzstoffen und gegebenenfalls mit einer oder mehreren kompatiblen und pharmakologisch aktiven Verbindungen enthält.
